(19) **Europäisches Patentamt European Patent Office Office européen des brevets**

(11) **EP 4 137 237 B1**

(12) **EUROPEAN PATENT SPECIFICATION**

(45) Date of publication and mention of the grant of the patent:
**15.01.2025 Bulletin 2025/03**

(21) Application number: **22190937.7**

(22) Date of filing: **18.08.2022**

(51) International Patent Classification (IPC):
**B05B 12/08** *(2006.01)* **B05B 17/06** *(2006.01)*
**A61M 11/00** *(2006.01)* **A61M 15/00** *(2006.01)*

(52) Cooperative Patent Classification (CPC):
**B05B 12/081; A61M 11/005; A61M 15/0085; B05B 17/0607;** A61M 2202/0468; A61M 2205/18; A61M 2205/3317; A61M 2205/3386; A61M 2205/3592; A61M 2205/50; A61M 2205/502; A61M 2205/505

(54) **NO-LOAD OPERATION DETECTION METHOD AND APPARATUS, NO-LOAD OPERATION PROTECTION METHOD AND APPARATUS, AND VAPORIZER**

LEERLAUFBETRIEBSDETEKTIONSVERFAHREN UND -GERÄT, LEERLAUFBETRIEBSSCHUTZVERFAHREN UND -GERÄT UND VERDAMPFER

PROCÉDÉ ET APPAREIL DE DÉTECTION DE FONCTIONNEMENT À VIDE, PROCÉDÉ ET APPAREIL DE PROTECTION DE FONCTIONNEMENT À VIDE, ET VAPORISATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2021 CN 202110960632**

(43) Date of publication of application:
**22.02.2023 Bulletin 2023/08**

(73) Proprietor: **Shenzhen Moore Vaporization Health & Medical Technology Co., Ltd. Guangdong 401 (CN)**

(72) Inventor: **Yang, Sheng Shenzhen 401 (CN)**

(74) Representative: **Michalski Hüttermann & Partner Patentanwälte mbB Kaistraße 16A 40221 Düsseldorf (DE)**

(56) References cited:
**EP-A1- 2 554 514       EP-A1- 3 831 228 WO-A1-2014/062175**

**Description**

FIELD

This application relates to the field of vaporization technologies, and in particular, to a no-load operation detection method and apparatus and a no-load operation protection method and apparatus applied to a vaporizer, a vaporizer, a computer device, and a storage medium.

BACKGROUND

With the continuous improvement of living standards, the medical aesthetics industry is developing faster and faster, and vaporizers can help improve the aesthetics effect. For example, when a vaporizer is used to perform cold vaporization on an essence, the essence may be distributed at 100% and active ingredients in the essence may be maintained, so that the essence can be more easily absorbed by the human body, thereby exerting the maximum effect.

During use, the vaporizer can be normally vaporized only when there is the essence, and a problem of no-load operation occurs when there is a lack of essence. To prevent no-load operation, the existing vaporizer generally uses an electrode method to detect whether there is a lack of essence. A principle of the method is that an electrode is connected to a negative electrode of a vaporization piece through the essence, and whether there is a lack of essence in the vaporizer is determined by detecting an electrical change of the electrode.

The existing vaporizer needs to introduce the electrode to detect whether there is a lack of essence to prevent no-load operation. Affected by the structure, material, and hardware circuit, etc., the implementation effect is poor. The document EP 2 554 514 A1 discloses a hydrogen peroxide gas production device being provided with an atomization unit which atomizes hydrogen peroxide stored in a storage part by applying ultrasonic vibration to the hydrogen peroxide, a heater which is provided above the atomization unit and heats the hydrogen peroxide atomized by the atomization unit to gasify the hydrogen peroxide, a metallic internal cylinder part in which the heater is disposed in the inner space thereof and which guides upward the hydrogen peroxide that is atomized by the atomization unit and flows together with carrier gas and an external cylinder part in which the internal cylinder part is disposed in the inner space thereof to configure a double tube and which forms, with the internal cylinder part, a gas flow path for the carrier gas that goes down toward the storage part therebetween. The device is characterized by being configured so that the carrier gas flowing through the gas flow path is brought into contact with the internal cylinder part heated by the heater and the heated carrier gas is introduced into the storage part. EP 3 831 228 A1 discloses an aerosol generating device for generating aerosol inhaled by a user, and a method and a program for operating the aerosol generating device.

SUMMARY

In an embodiment, the present application provides a no-load operation detection method, applied to a vaporizer, the method comprising: obtaining vaporization parameters of a vaporization piece in real time, and calculating a variance between the vaporization parameters and a sample mean, the sample mean being a value representing a vaporization parameter level of a stable vaporization stage of the vaporizer; and determining that the vaporization piece is operated without load if the variance is greater than a preset variance value corresponding to the vaporization parameter, the preset variance value being used for distinguishing a vaporization parameter fluctuation caused by vaporization operation without load and a vaporization parameter fluctuation caused by another operation other than vaporization without load, wherein the preset variance value is obtained by: obtaining vaporization parameters when the vaporization piece is no-load operation, and calculating a no-load operation signal variance corresponding to the vaporization parameters when the vaporization piece is no-load operation ; obtaining vaporization parameters of the vaporization piece when to-be-vaporized liquid is triggered to generate bubbles, and calculating a bubble signal variance corresponding to the vaporization parameters of the vaporization piece generated at the moment when the to-be-vaporized liquid is triggered to generate bubbles; and selecting a value greater than the bubble signal variance and less than the no-load operation signal variance as the preset variance value.

BRIEF DESCRIPTION OF THE DRAWINGS

Subject matter of the present application will be described in even greater detail below based on the exemplary figures. All features described and/or illustrated herein can be used alone or combined in different combinations. The features and advantages of various embodiments will become apparent by reading the following detailed description with reference to the attached drawings, which illustrate the following:

FIG. 1 is a schematic structural diagram of a vaporizer according to an embodiment;

FIG. 2 is a schematic flowchart of a no-load operation detection method according to an embodiment;

FIG. 3 is a schematic flowchart of a no-load operation detection method according to another embodiment;

FIG. 4a(1) is a schematic diagram of a vaporization current generated by an influence of bubbles according to an embodiment;

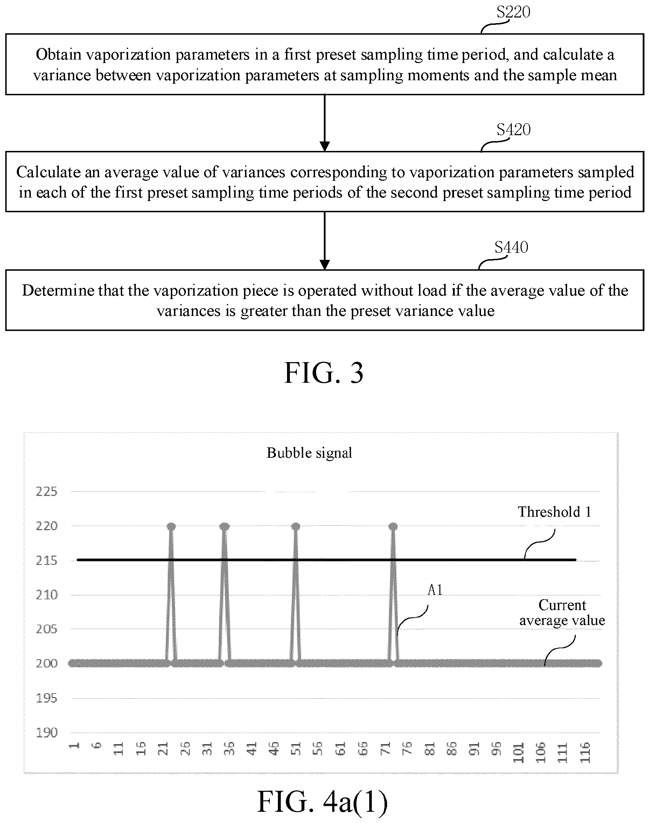

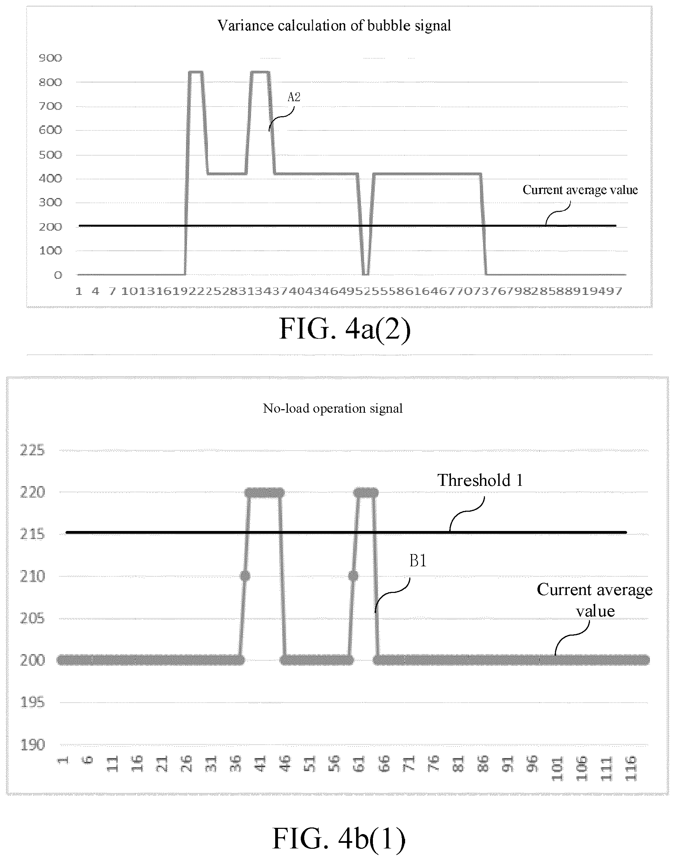

FIG. 4a(2) is a schematic diagram of a signal of the vaporization current in FIG. 4a(1) after variance calculation;

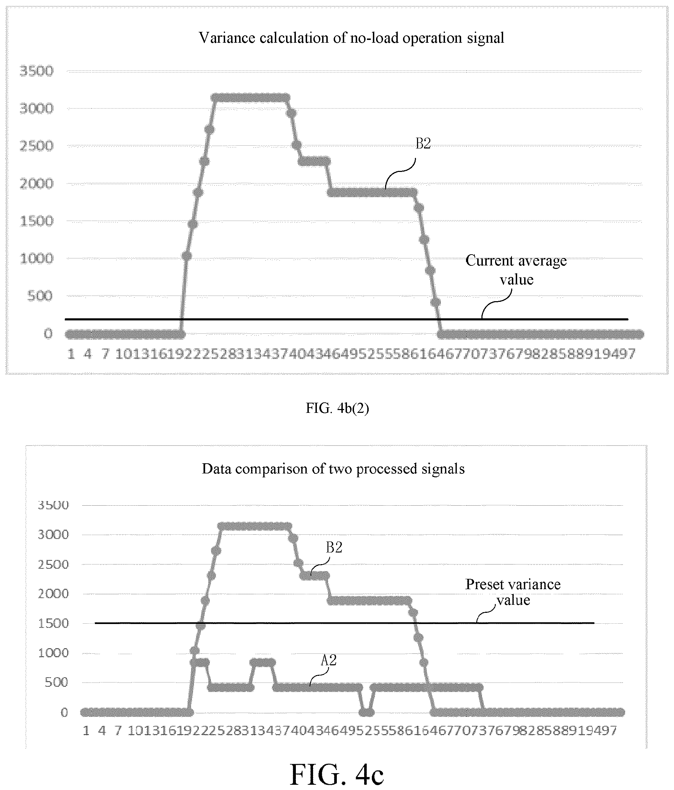

FIG. 4b(1) is a schematic diagram of a vaporization current generated by being influenced by no-load operation of a vaporization piece according to an embodiment;

FIG. 4b(2) is a schematic diagram of a signal of the vaporization current in FIG. 4b(1) after variance calculation;

FIG. 4c is a schematic diagram of comparison of the signals of the vaporization currents after variance calculation in FIG. 4a(2) and FIG. 4b(2);

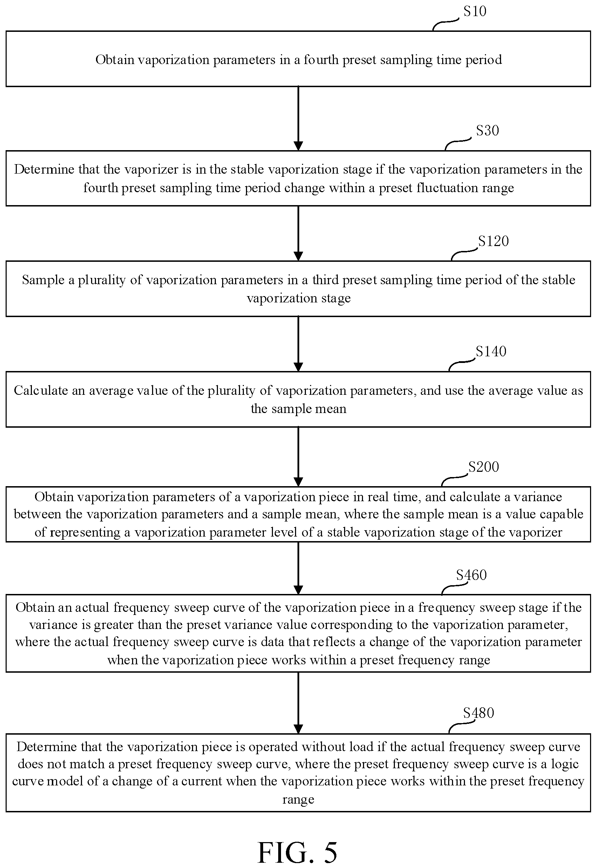

FIG. 5 is a schematic flowchart of a no-load operation detection method according to another embodiment;

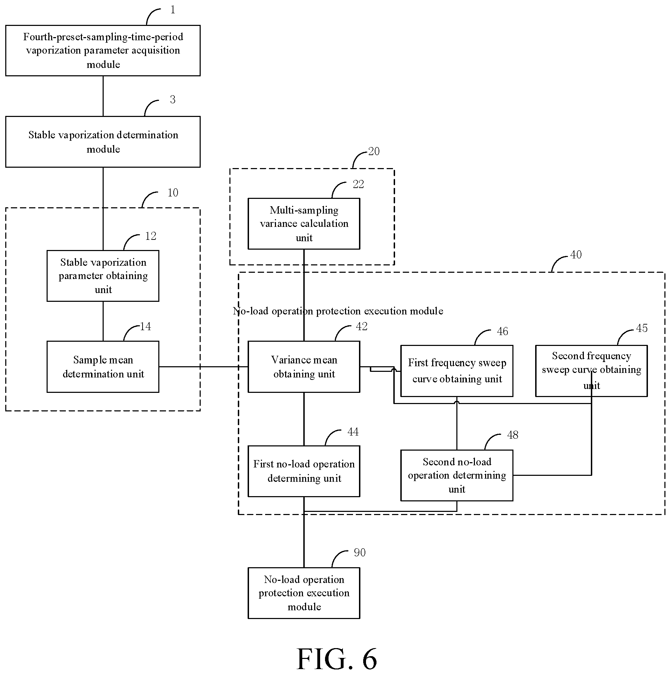

FIG. 6 is a structural block diagram of a no-load operation detection apparatus and a no-load operation protection apparatus according to an embodiment;

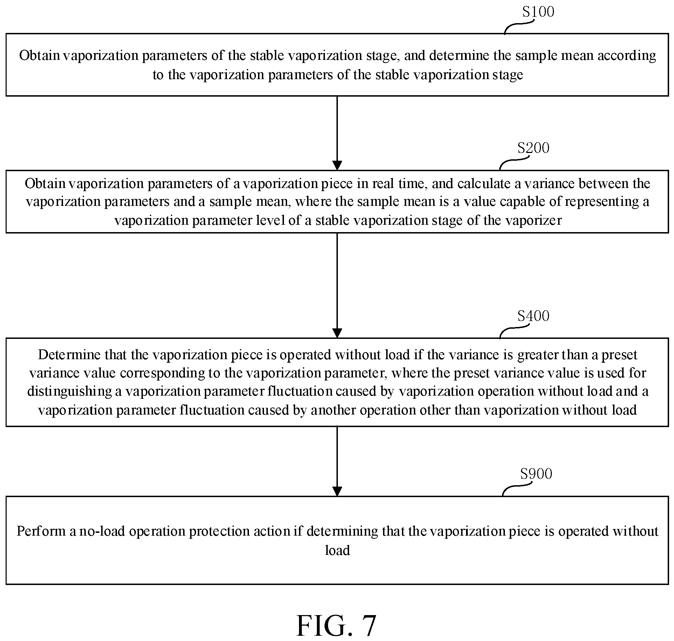

FIG. 7 is a schematic flowchart of a no-load operation protection method according to an embodiment;

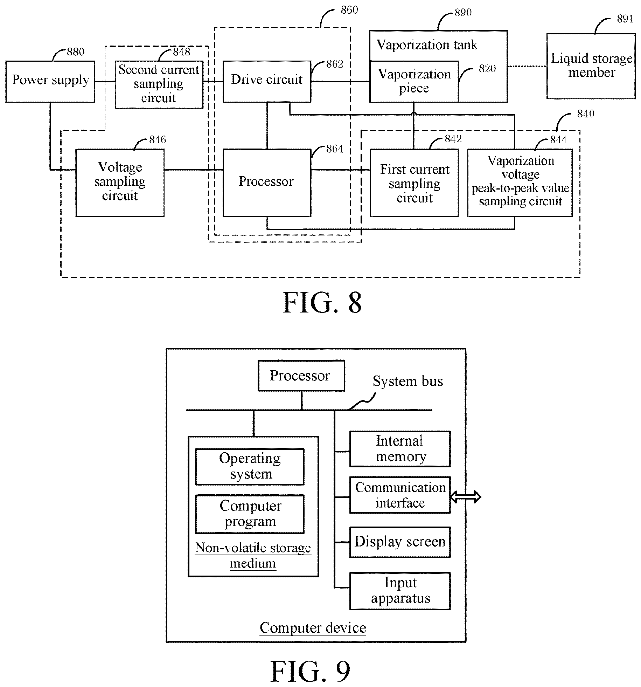

FIG. 8 is a schematic structural diagram of a vaporizer according to another embodiment; and

FIG. 9 is a block diagram of an internal structure of a computer device according to an embodiment.

DETAILED DESCRIPTION

In an embodiment, the present application provides a high-precision no-load operation detection method and apparatus that are simple to implement and can eliminate an influence of bubbles, a vaporizer, and a computer device.

An aspect of embodiments of this application provides a no-load operation detection method, applied to a vaporizer, the method including:

obtaining vaporization parameters of a vaporization piece in real time, and calculating a variance between the vaporization parameters and a sample mean, where the sample mean is a value capable of representing a vaporization parameter level of a stable vaporization stage of the vaporizer; and

determining that the vaporization piece is operated without load if the variance is greater than a preset variance value corresponding to the vaporization parameter, where the preset variance value is used for distinguishing a vaporization parameter fluctuation caused by vaporization operation without load and a vaporization parameter fluctuation caused by another operation other than vaporization without load.

According to the no-load operation detection method provided in the embodiments of this application, vaporization parameters of a vaporization piece are obtained, a corresponding variance is calculated based on the vaporization parameters, and a value of the variance is compared with a preset variance value. If the value of the variance is greater than the preset variance value, it is determined that the vaporization parameter changes greatly at this time due to no-load operation of the vaporization piece, that is, the vaporization piece has a phenomenon of no-load operation. The implementation process does not require additional hardware, and the implementation is fast and convenient. Moreover, in full consideration of the influence of factors such as characteristics and a structure of a liquid sol medium itself such as an essence, bubbles may be generated in a vaporization process, and the vaporization parameter may also fluctuate at the moment when the bubbles are generated. For this interference factor, in the solution provided in the embodiments of this application, a difference between a vaporization parameter fluctuation triggered by the bubbles and a vaporization parameter fluctuation triggered by no-load operation is increased by using the above variance calculation manner; a value between a vaporization parameter variance corresponding to the condition other than vaporization operation without load and a vaporization parameter variance corresponding to the condition of vaporization operation without load is selected as the preset variance value; and the variance corresponding to the actually obtained vaporization parameter is compared with the preset variance value during no-load operation detection. In this way, no-load operation determination can be quickly implemented, and the detection implementation solution well eliminates the interference of the vaporization parameter fluctuation on a detection result caused in the condition other than vaporization operation without load, thereby improving the recognition accuracy of vaporization operation without load.

In an embodiment, the step of obtaining vaporization parameters of a vaporization piece in real time, and calculating a variance between the vaporization parameters and a sample mean includes:

obtaining vaporization parameters in a first preset sampling time period, and calculating a variance between vaporization parameters at sampling moments and the sample mean; and

a second preset sampling time period includes a plurality of independent first preset sampling time periods, and the step of determining that the vaporization piece is operated without load if the variance is greater than a preset variance value corresponding to the vaporization parameter includes:

calculating an average value of variances corresponding to vaporization parameters sampled in each of the first preset sampling time periods of the second preset sampling time period; and

determining that the vaporization piece is operated without load if the average value of the variances is greater than the preset variance value.

In an embodiment, the vaporization parameter includes a vaporization current and/or vaporization voltage peak-to-peak value and/or vaporization power.

In an embodiment, the step of determining that the vaporization piece is operated without load if the variance is greater than a preset variance value corresponding to the vaporization parameter includes:

obtaining an actual frequency sweep curve of the vaporization piece in a frequency sweep stage if the variance is greater than the preset variance value corresponding to the vaporization parameter, where the actual frequency sweep curve is data that reflects a change of the vaporization parameter when the vaporization piece works within a preset frequency range; and

determining that the vaporization piece is operated without load if the actual frequency sweep curve does not match a preset frequency sweep curve, where the preset frequency sweep curve is a logic curve model of a change of a current when the vaporization piece works within the preset frequency range.

In an embodiment, the no-load operation detection method further includes:
obtaining vaporization parameters of the stable vaporization stage, and determining the sample mean according to the vaporization parameters of the stable vaporization stage.

In an embodiment, the step of obtaining vaporization parameters of the stable vaporization stage, and determining the sample mean according to the vaporization parameters of the stable vaporization stage includes:
sampling a plurality of vaporization parameters in a third preset sampling time period of the stable vaporization stage; and calculating an average value of the plurality of vaporization parameters, and using the average value as the sample mean.

In an embodiment, before the step of obtaining vaporization parameters of the stable vaporization stage, the method further includes: obtaining vaporization parameters in a fourth preset sampling time period; and

determining that the vaporizer is in the stable vaporization stage if the vaporization parameters in the fourth preset sampling time period change within a preset fluctuation range. Another aspect of the embodiments of this application further provides a no-load operation detection apparatus, applied to a vaporizer, the apparatus including:

a variance calculation module, configured to obtain vaporization parameters of a vaporization piece in real time, and calculate a variance between the vaporization parameters and a sample mean, where the sample mean is a value capable of representing a vaporization parameter level of a stable vaporization stage of the vaporizer; and

a no-load operation determination module, configured to determine that the vaporization piece is operated without load if the variance is greater than a preset variance value, where the preset variance value is used for distinguishing a vaporization parameter fluctuation caused by vaporization operation without load and a vaporization parameter fluctuation caused by another operation other than vaporization without load.

The embodiments of this application further provide a vaporizer, including: a vaporization piece, configured to vaporize to-be-vaporized liquid;

a sampling circuit, configured to sample vaporization parameters of the vaporization piece; and

a controller, where the controller is electrically connected to the vaporization piece and the sampling circuit respectively, and is configured to drive the vaporization piece to vibrate, so that the vaporization piece vaporizes the to-be-vaporized liquid; and the controller is further configured to perform the steps of the no-load operation detection method and/or the steps of the no-load operation protection method.

In an embodiment, the controller in the vaporizer includes:

a drive circuit, configured to drive the vaporization piece to vibrate, so that the vaporization piece vaporizes the to-be-vaporized liquid; and

a processor, connected to the drive circuit, and configured to regulate a signal outputted by the drive circuit to the vaporization piece.

In an embodiment, the vaporization parameter includes a vaporization current and/or vaporization voltage peak-to-peak value and/or vaporization power; and the sampling circuit includes:

a first current sampling circuit, where an input end of the first current sampling circuit is electrically connected to the vaporization piece, an output end of the first current sampling circuit is connected to the processor, and the first current sampling circuit is configured to sample the vaporization current; and/or

a vaporization voltage peak-to-peak value sampling circuit, where an input end of the vaporization voltage peak-to-peak value sampling circuit is connected to an output end of the drive circuit, an output end of the vaporization voltage peak-to-peak value sampling circuit is connected to the processor, and the vaporization voltage peak-to-peak value sampling circuit is configured to sample the vaporization voltage peak-to-peak value; and/or

a voltage sampling circuit and a second current sampling circuit, where an input end of the voltage sampling circuit is connected to a vaporization power supply, an output end of the voltage sampling circuit is connected to the processor, and the voltage sampling circuit is configured to sample a vaporization input voltage; an input end of the second current sampling circuit is configured to be connected in series between the power supply and the drive circuit, and the second current sampling circuit is configured to sample a vaporization input current; and the processor is further configured to calculate the vaporization power according to the vaporization input voltage and the vaporization input current.

In an embodiment, the vaporizer further includes:

a vaporization tank, where the vaporization tank is configured to accommodate the to-be-vaporized liquid, and the vaporization piece is arranged in the vaporization tank; and

a liquid storage member, where a liquid storage cavity is formed in the liquid storage member, the liquid storage cavity is configured to store the to-be-vaporized liquid, and the liquid storage cavity is in communication with the vaporization tank.

To help understand this application, this application is described more fully with reference to the related accompanying drawings in the following. The accompanying drawings show embodiments of this application. However, this application may be implemented in many different forms, and is not limited to the embodiments described in this specification. On the contrary, the embodiments are provided to make the disclosed content of this application more thorough and comprehensive.

Unless otherwise defined, meanings of all technical and scientific terms used in this specification are the same as those usually understood by a person skilled in the art to which this application belongs. In this specification, terms used in the specification of this application are merely intended to describe objectives of the specific embodiments, but are not intended to limit this application.

It may be understood that, terms "first", "second" and the like used in this specification may be used for describing various time periods, but these time periods are not limited by these terms. The terms are merely used for distinguishing a first time period from another time period.

It should be noted that, when an element is considered to be "connected" to another element, the element may be directly connected to the other element, or may be electrically connected to the another element through a central element. In addition, the "connection" in the following embodiments should be understood as "electrical connection", "communication connection", and the like if there is transmission of electrical signals or data between the connected objects.

As used herein, terms "a", "one", and "the" which are singular forms may also include plural forms, unless otherwise specified in the context clearly. It should further be understood that terms "comprise/include" and/or "contain" and the like indicate the presence of features, integers, steps, operations, components, parts, and/or combinations thereof, but do not preclude the possibility of the presence or addition of one or more other features, integers, steps, operations, components, parts, and/or combinations thereof. At the same time, a term "and/or" used in this specification includes any or all combinations of related listed items.

For the problems provided in the background, in an exemplary technology, an upper limit threshold and a lower limit threshold are set by obtaining an average vaporization parameter of a vaporization piece during normal vaporization. In a subsequent working process of a vaporizer, whether the vaporization piece is operated without load is determined by monitoring whether an actual vaporization parameter of the vaporization piece exceeds the upper limit threshold and the lower limit threshold. If the vaporization parameter exceeds a range determined by the upper limit threshold and the lower limit threshold, it is determined that the vaporization piece is operated without load.

However, the inventor found during the implementation process that, in an aesthetics vaporization project, due to the influence of factors such as characteristics, a structure of an essence itself, bubbles may be generated during a vaporization process. In this case, the vaporization parameter (such as a current, power, and a voltage peak-to-peak value of the vaporization piece) may have a relatively large increase or decrease at the moment when the bubbles are generated. When the bubbles disappear, the vaporization parameter returns to a normal working level. As a result, when the bubbles are generated, an instantaneous value of the vaporization parameter may exceed the range determined by the set upper limit threshold and the lower limit threshold, and the frequent generation of the bubbles during the vaporization process easily causes false recognition of no-load operation detection, that is, a detection result for vaporization operation without load has low reliability.

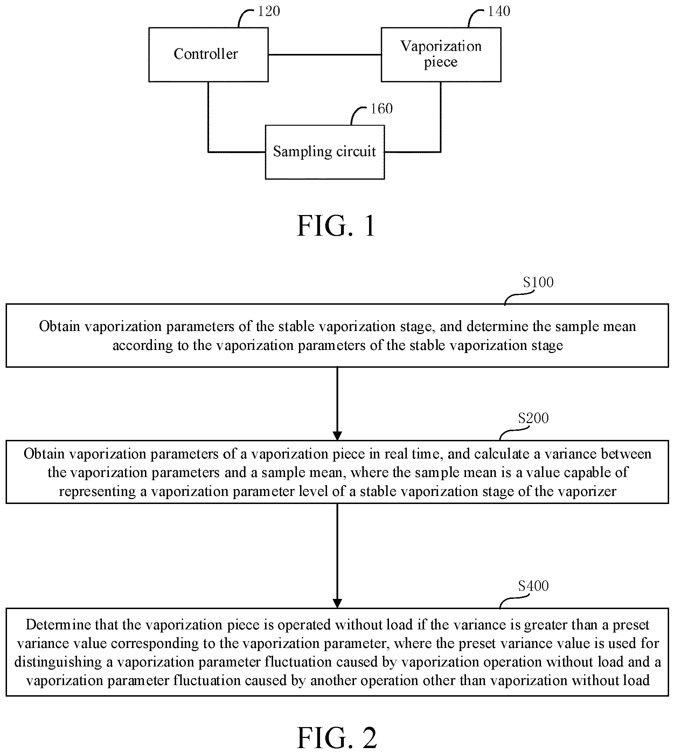

For this, in an embodiment, a no-load operation detection method is provided, and the method may be applied to a vaporizer shown in FIG. 1. To implement vaporization control, the vaporizer generally includes a controller 120, a vaporization piece 140, and a sampling circuit 160. The controller 120 is configured to output a pulse width modulation (PWM) drive signal to drive the vaporization piece 140 to work. The vaporization piece 140 is configured to vaporize to-be-vaporized liquid (such as medicinal liquid, an essence, etc.) under the driving of the controller 120. The sampling circuit 160 is configured to acquire and feed back, to the controller 120, at least one of parameters such as a current flowing through the vaporization piece 140, a peak-to-peak value of a voltage of the controller 120 acting on the vaporization piece 140, and input power provided by a power supply of the vaporizer for vaporization, so that the controller 120 regulates the outputted PWM drive signal according to the vaporization parameter. It can be understood by a person skilled in the art that, FIG. 1 only shows main components for implementing vaporization control. According to functional requirements of the vaporizer, more components may be included. FIG. 1 is only for reference, to facilitate the understanding of the no-load operation detection method and the no-load operation protection method in the following embodiments, and does not limit the formation of the vaporizer for implementing the corresponding methods.

In an embodiment, as shown in FIG. 2, W' the no-load operation detection method includes the following steps.

S200: Obtain vaporization parameters of a vaporization piece in real time, and calculate a variance between the vaporization parameters and a sample mean, where the sample mean is a value capable of representing a vaporization parameter level of a stable vaporization stage of the vaporizer.

The vaporizer is a device that can vaporize to-be-vaporized liquid. The vaporization piece may vibrate under the driving of an electric signal, and a molecular structure of the to-be-vaporized liquid in contact with the vaporization piece is broken up by using the high-frequency vibration, to generate fine mist droplets. A change of a vaporization parameter in a working process of the vaporizer compared with a vaporization parameter when the vaporizer works normally is detected, so that the determination of vaporization operation without load can be finally implemented, that is, the vaporization parameter when the vaporizer works normally provides a very important reference for no-load operation detection. Based on this, by obtaining the vaporization parameter of the stable vaporization stage of the vaporizer, a value that can reflect a vaporization parameter level when the vaporizer works normally is determined as the sample mean, to provide a data basis for no-load operation detection.

S400: Determine that the vaporization piece is operated without load if the variance is greater than a preset variance value corresponding to the vaporization parameter, where the preset variance value is used for distinguishing a vaporization parameter fluctuation caused by vaporization operation without load and a vaporization parameter fluctuation caused by another operation other than vaporization without load.

The variance is a variance corresponding to the vaporization parameters, namely, a mean of a sum of squares of deviations of vaporization parameters sampled in real time and the sample mean. For example, if n vaporization parameters are actually sampled during no-load operation detection, a variance $s^2$ corresponding to the vaporization parameters may be calculated according to the following formula:

$$s^2 = \frac{(x_1 - M)^2 + (x_2 - M)^2 + (x_3 - M)^2 + ... + (x_n - M)^2}{n}$$

$x_1, x_2, ..., x_n$ are vaporization parameters obtained by $n^{th}$ sampling, where n is greater than or equal to 1. M is the sample mean. The variance may be used for describing a degree of deviation of the actual vaporization parameters from the sample mean. Based on research and testing, the inventor found that the degree of deviation of the vaporization parameters from the sample mean during no-load operation is larger than that of vaporization parameters during another operation other than vaporization without load. The detection for vaporization operation without load is implemented by using this difference.

The vaporization parameter fluctuation caused by the vaporization operation without load is a vaporization parameter fluctuation caused when the vaporization piece is operated without load. The vaporization parameter fluctuation caused by another operation other than vaporization without load is a vaporization parameter fluctuation caused by factors other than no-load operation of the vaporization piece, for example, a change of the vaporization parameter caused at the moment when an essence generates bubbles when the vaporization piece vaporizes the essence.

Specifically, when a vaporizer works, vaporization parameters of a vaporization piece are obtained in real time, a corresponding variance is calculated based on the vaporization parameters, and a value of the variance is compared with a preset variance value. If the value of the variance is greater than the preset variance value, it is determined that the vaporization parameter changes greatly at this time due to no-load operation of the vaporization piece, that is, the vaporization piece has a phenomenon of no-load operation. The implementation process does not require additional hardware, and the implementation is fast and convenient. Moreover, in full consideration of the influence of factors such as characteristics and a structure of a liquid sol medium itself such as an essence, bubbles may be generated in a vaporization process, and the vaporization parameter may also fluctuate at the moment when the bubbles are generated. For this interference factor, in the solution provided in the embodiments of this application, a difference between a vaporization parameter fluctuation triggered by the bubbles and a vaporization parameter fluctuation triggered by no-load operation is increased by using the above variance calculation manner; a value between a vaporization parameter variance corresponding to the condition other than vaporization operation without load and a vaporization parameter variance corresponding to the condition of vaporization operation without load is selected as the preset variance value; and the variance corresponding to the actually obtained vaporization parameter is compared with the preset variance value during no-load operation detection. In this way, no-load operation determination can be quickly implemented, and the detection implementation solution well eliminates the interference of the vaporization parameter fluctuation on a detection result caused in the non-vaporization no-load operation condition, thereby improving the recognition accuracy of vaporization operation without load.

Based on the accurate recognition result of the vaporization operation without load, a protection action such as no-load operation alarming or no-load operation shutdown can be reasonably performed, to avoid damage to various components in the vaporizer caused by long-term no-load operation or occurrence of safety accidents. According to the invention, the step of obtaining the preset variance value includes:

obtaining vaporization parameters when the vaporization piece is no-load operation, and calculating a no-load operation signal variance corresponding to the vaporization parameters when the vaporization piece is no-load operation;

obtaining vaporization parameters of the vaporization piece when to-be-vaporized liquid is triggered to generate bubbles, and calculating a bubble signal variance corresponding to the vaporization parameters of the vaporization piece generated at the moment when the to-be-vaporized liquid is triggered to generate bubbles; and

selecting a value greater than the bubble signal variance and less than the no-load operation signal variance as the preset variance value.

The to-be-vaporized liquid may be selected according to an actual application scene of the vaporizer, to measure a bubble signal variance when the vaporizer is actually used, thereby providing a more targeted preset variance value for the no-load operation detection during subsequent actual use of the vaporizer. For example, if the vaporizer is used in the aesthetics industry, the vaporizer is often used to vaporize the essence in common scenarios, and accordingly the essence may be selected as the to-be-vaporized liquid to perform the obtaining of bubble signal variance. In this way, when the vaporizer is used to vaporize the essence in an aesthetics salon, because the selected preset variance value is exactly a value determined for the to-be-vaporized liquid, the vaporizer can quickly and accurately determine whether there is no-load operation by performing the steps of the no-load operation detection method. Based on the accurate detection result, a no-load operation protection action such as no-load operation alarming or no-load operation shutdown can be more reasonably performed during no-load operation.

In an embodiment, at the time of determining the preset variance value, after a plurality of times of trials, a value greater than bubble signal variances obtained from the trials and less than no-load operation signal variances obtained from the trials is selected as the preset variance value, to avoid a problem that the preset variance value is unreasonably set due to contingency of a single trial.

In addition, in an embodiment, at the time of determining the preset variance value, according to to-be-vaporized liquid commonly used in the application scenario of the vaporizer, a plurality of times of trials of the above embodiment are performed for each type of the to-be-vaporized liquid, and bubble signal variances and no-load operation signal variances when each type of the to-be-vaporized liquid is trialed are obtained, to determine a value that can meet a condition of being greater than the bubble signal variances obtained from the trials of various types of to-be-vaporized liquid and less than the no-load operation signal variances obtained from the trials of various types of to-be-vaporized liquid in this scenario as the preset variance value. In this way, the vaporizer performing the no-load operation detection method can accurately implement no-load operation determination for different types of to-be-vaporized liquid when being used in a selected application scenario. For example, when the vaporizer is used in the aesthetic salon, the preset variance value is determined by testing and obtaining no-load operation signal variances and bubble signal variances of different essence products in test processes, which can ensure that the vaporizer can implement accurate no-load operation detection of the vaporization piece by performing the steps of the no-load operation detection method when the aesthetic salon provides essences of different brands for different users.

In an embodiment, as shown in FIG. 3, the step S200 of obtaining vaporization parameters of a vaporization piece in real time, and calculating a variance between the vaporization parameters and a sample mean includes the following step.

5220: Obtain vaporization parameters in a first preset sampling time period, and calculate a variance between vaporization parameters at sampling moments and the sample mean. The setting of the first preset sampling time period may be correspondingly configured according to a specific application scenario, for example, 15s. The vaporization parameter in the vaporization process may be continuously obtained. However, to improve the calculation efficiency, a plurality of sampling points may be set in the first preset sampling time period, and a variance calculation may be performed according to data acquired by the sampling points, to improve the calculation efficiency, thereby improving the no-load operation determination efficiency.

A second preset sampling time period includes a plurality of independent first preset sampling time periods, and the step S400 of determining that the vaporization piece is operated without load if the variance is greater than a preset variance value corresponding to the vaporization parameter includes the following steps.

S420: Calculate an average value of variances corresponding to vaporization parameters sampled in each of the first preset sampling time periods of the second preset sampling time period. The second preset sampling time period includes at least one first preset sampling time period. For example, the second preset sampling time period may include four first preset sampling time periods, the second preset sampling time period is 40s, each first preset sampling time period is 8s, a time interval between two adjacent first preset sampling time periods is 2s, and in each first preset sampling time period, the sampling is performed every two seconds, for a total of four times of sampling.

S440: Determine that the vaporization piece is operated without load if the average value of the variances is greater than the preset variance value.

According to the no-load operation detection method provided in this embodiment of this application, a plurality of times of sampling are performed in the first preset sampling time period, and variances of sampled vaporization parameters in the first preset sampling time period are calculated, to thereby eliminate a problem of unreliable detection caused by contingency of single measurement. Moreover, by performing at least one time of sampling in the second preset sampling time period, for example, four times of parameter sampling in the first preset sampling time period as described above, and synthesizing the plurality of variances obtained in the second preset sampling time period, an average value of the variances is calculated, which can avoid false detection caused by occurrence of accidental events in the first preset sampling time period. Finally, comparison is performed based on the average value of the variances and a preset variance value. If the average value is greater than the preset variance value, it is determined that the vaporization piece is operated without load. Based on this design concept, the vaporizer performing the steps of the no-load operation detection method can accurately implement no-load operation detection of the vaporization piece, and perform a no-load operation protection action when it is determined that the vaporization piece is operated without load. For example, the no-load operation protection action may include sound and light alarming, power-off protection, and the like.

In an embodiment, the vaporization parameter includes a vaporization current, a vaporization voltage peak-to-peak value or vaporization power. The vaporization current is a current signal flowing through the vaporization piece. The vaporization voltage peak-to-peak value is a peak-to-peak value of a voltage signal inputted to the vaporization piece. The vaporization power is power corresponding to an input voltage and an input current provided for the vaporizer by a power supply that powers the vaporizer. When different vaporization parameters are selected, the preset variance value also changes accordingly. For example, when the vaporization current is selected as the vaporization parameter for no-load operation detection, the preset variance value corresponding to the vaporization parameter is a preset value corresponding to the vaporization current, that is, the preset variance value is a value capable of distinguishing a vaporization current fluctuation caused by vaporization operation without load and a vaporization current fluctuation caused by another operation other than vaporization without load. Correspondingly, when the preset variance value is determined by using the steps described in the above embodiments, the sampled vaporization parameter is also the vaporization current.

In an embodiment, the step of determining that the vaporization piece is operated without load if the variance is greater than a preset variance value includes: determining that the vaporization piece is operated without load if a variance corresponding to any type of vaporization parameter is greater than the preset variance value.

In an embodiment, the step of determining that the vaporization piece is operated without load if the average value of the variances is greater than the preset variance value includes:
determining that the vaporization piece is operated without load if an average value of variances corresponding to any type of vaporization parameter is greater than the preset variance value. When there are a plurality of vaporization parameters, it may be determined that the vaporization piece is operated without load when a variance or an average value of variances corresponding to any type of vaporization parameter is greater than the preset variance value, which can ensure that the validity of no-load operation detection can still be ensured when an obtaining channel of any type of vaporization parameter fails, so that the reliability of no-load operation detection is improved, and the no-load operation protection implemented based on the no-load operation detection method is more reliable.

To better illustrate the implementation process of the no-load operation detection method provided in the embodiments of this application, an example in which the vaporization parameter is the vaporization current is used for description. It should be noted that, the example herein does not limit the actual protection scope of this application. A variance between sampled vaporization currents in a time period and a sample mean (namely, a current average value) is calculated, where the current average value is a mean of currents sampled in a time period when the vaporization is in a stable state. After current mean sampling is completed, a variance between subsequently sampled currents and the current average value is calculated.

As shown in FIG. 4a(1) and FIG. 4b(1), if a threshold 1 is selected for no-load operation determination, two curves A1 and B1 are respectively curves of the bubble signal and the no-load operation signal, and change ranges of the two curves have little difference. If a current threshold is used for determining (that is, the threshold 1 in the figure is used for determining) whether there is vaporization operation without load, because both the two signals exceed the threshold 1, when the bubble signal causes this fluctuation, it is also determined as no-load operation, resulting in false detection.

As shown in FIG. 4a(2) and FIG. 4b(2), curves A2 and B2 are correspondingly obtained after respectively calculating variances of the bubble signal A1 and the no-load operation signal B1, and FIG. 4c is obtained by comparing two conditions of A2 and B2. As can be seen from a curve C1 in FIG. 4c, a difference between change ranges of vaporization current variances of A2 and B2 has a great improvement (for example, more than thrice is reached in C1) compared with that of the vaporization current curves. Through actual detected data, a value between two ranges may be set as the preset variance value, thereby accurately recognizing that the signal A1 is the bubble signal and the signal B1 is the no-load operation signal, that is, it may be determined whether the vaporization parameter fluctuation is caused by vaporization operation without load, and it is determined that the vaporization piece is operated without load when the variance is greater than the preset variance value.

An aesthetic vaporizer is used as an example. By comparison, under a condition that change ranges of currents are substantially the same, a changed vaporization current caused by no-load operation is referred to as a no-load operation signal herein, and it is obtained through a calculation that a range of a variance curve of the no-load operation signal has a great improvement (for example, more than twice may be reached, and more than thrice is further reached in FIG. 4c) compared with a variance curve of a changed vaporization current (referred to as the a bubble signal herein) caused at the moment when essence is vaporized and generates bubbles, so that the no-load operation signal and the bubble signal are distinguished more obviously, thereby improving the accuracy of system no-load operation recognition.

The specific working implementation process is as follows. After vaporization of the vaporizer is stable, vaporization currents are sampled (for example, 20 pieces of data may be acquired in a third preset sampling time period), and an average value $I_0$ thereof is calculated as a current sample mean for variance calculation. For example, a vaporization current fluctuation in a time period may be detected, and the vaporization is considered to be stable if the fluctuation range is steady. For example, for a conventional vaporizer, a fluctuation range does not exceed $\pm 5\%$ when there is no bubble in normal. Therefore, when the vaporization current fluctuation does not exceed the range of $\pm 5\%$, it may be determined that the vaporizer enters a stable vaporization stage, and a sample mean is calculated according to sampled vaporization currents in this stage.

In an actual use process of the vaporizer, a vaporization current $I_{nk}$ is continuously sampled, and a variance $P_{nk}$ of vaporization currents at sampling points is calculated. The calculation formula is as follows:

$$P_{nk} = \frac{\sum (I_{nk} - I_0)^2}{n}$$

$P_{nk}$ represents a value of a variance obtained by sampling n vaporization parameters in a $k^{th}$ first preset sampling time period, $I_{nk}$ represents a vaporization current at an $n^{th}$ sampling point in the $k^{th}$ first preset sampling time period, and n = 1, 2, ..., N, where N is a number of times of sampling in a single first preset sampling time period, for example, N may be equal to 20.

For a plurality of pieces of variance data (for example, k pieces of variance data) obtained in the second preset sampling time period, an average value $P_v$ thereof may be further calculated, and the calculation formula is as follows:

$$P_v = \frac{\sum p_{nk}}{k}$$

When the average value $P_v$ of the variances exceeds a preset variance value, it is determined that the vaporization piece is in a state of no-load operation.

For implementation processes of other types of vaporization parameters such as a vaporization voltage peak-to-peak value and vaporization power, reference may be made to the above explanation of the vaporization current for understanding, and details are not described herein.

In an embodiment, as shown in FIG. 5, to further improve the accuracy and reliability of the no-load operation detection result, the step S400 of determining that the vaporization piece is operated without load if the variance is greater than a preset variance value corresponding to the vaporization parameter includes the following steps.

S460: Obtain an actual frequency sweep curve of the vaporization piece in a frequency sweep stage if the variance is greater than the preset variance value corresponding to the vaporization parameter, where the actual frequency sweep curve is data that reflects a change of the vaporization parameter when the vaporization piece works within a preset frequency range. The frequency sweep stage is a prepositive working stage for obtaining a resonant frequency of the vaporization piece. The resonant frequency of the vaporization piece is determined by controlling the vaporization piece to work at different frequencies according to the preset frequency range, so that the vaporization piece can obtain a maximum vaporization amount when working at the resonant frequency.

S480: Determine that the vaporization piece is operated without load if the actual frequency sweep curve does not match a preset frequency sweep curve, where the preset frequency sweep curve is a logic curve model of a change of a current when the vaporization piece works within the preset frequency range. The preset frequency sweep curve is a logic curve model of a change of a current flowing through the vaporization piece when the vaporization piece works at different frequencies in the preset frequency range in a normal working state without no-load operation. The logic curve model is a model for predicting a change trend of a curve, and contains determination and recognition of logic of curve features and data, and a general profile of the curve may be obtained by these features.

Because the current flowing through the vaporization piece may change in a state of no-load operation and a state other than no-load operation, and in order to further improve the reliability of the no-load operation detection and determination result provided in the embodiments of this application, when a variance or a mean of variances corresponding to the actual vaporization parameters is greater than the preset variance value, the actual frequency sweep curve and the preset frequency sweep curve are further compared. If the actual frequency sweep curve does not match the preset frequency sweep curve, it is determined that there is no-load operation; and otherwise, it is determined that the vaporization piece is not operated without load, and the vaporizer still remains normal working. Through the setting of the dual conditions, the accuracy of the detection result of the no-load operation detection method is further improved.

In an embodiment, a preset frequency sweep curve may be established by using a starting point, an uptrend, and a downtrend. The preset frequency sweep curve is a set of a series of curves with a same profile, rather than a fixed curve.

In an embodiment, as shown in FIG. 2, the no-load operation detection method further includes the following step.

S 100: Obtain vaporization parameters of the stable vaporization stage, and determine the sample mean according to the vaporization parameters of the stable vaporization stage. The vaporization parameters of the stable vaporization stage are obtained and are averaged to obtain the sample mean, and then based on the variance obtained from the sample mean, a degree of deviation of actual vaporization parameters from vaporization parameters when the vaporizer normally works may be accurately reflected, thereby further ensuring the accuracy of the no-load operation detection and determination result.

In an embodiment, as shown in FIG. 5, the step S100 of obtaining vaporization parameters of the stable vaporization stage, and determining the sample mean according to the vaporization parameters of the stable vaporization stage includes the following steps.

S 120: Sample a plurality of vaporization parameters in a third preset sampling time period of the stable vaporization stage. The third preset sampling time period is a time period in the stable vaporization stage, and the time period may be adaptively selected by pre-configuring a number of times of sampling and a sampling frequency. For example, when the sampling is configured in a manner that a time interval between adjacent two times of sampling is 1S and a total number of times of sampling is 15, the third preset sampling time period may be a time period from when it is determined to enter the stable vaporization stage to when a 15th sampling is completed. In the third preset sampling time period, the vaporization parameters may be sampled at equal time intervals.

S140: Calculate an average value of the plurality of vaporization parameters, and use the average value as the sample mean.

According to the no-load operation detection method provided in the embodiments of this application, by reasonably setting the third preset sampling time period, the reliability of the calculation result of the sample mean may be ensured, and the detection efficiency may be improved.

In an embodiment, as shown in FIG. 5, before the step S100 of obtaining vaporization parameters of the stable vaporization stage, the method further includes the following steps.

S10: Obtain vaporization parameters in a fourth preset sampling time period. Before the vaporizer is stable, the vaporization parameters thereof may fluctuate. For example, when the vaporizer is just started, in order to avoid that the sample mean calculated based on the vaporization parameters of this time period cannot accurately reflect parameter conditions of the vaporizer when working stably, the vaporization parameters in a time period may be acquired, and whether the vaporizer is in a stable working state may be determined by observing the change of the vaporization parameters.

S30: Determine that the vaporizer is in the stable vaporization stage if the vaporization parameters in the fourth preset sampling time period change within a preset fluctuation range.

The preset fluctuation range may be set correspondingly according to different specific selections of the vaporization piece. For example, in an implementation, if the vaporization current is selected as the vaporization parameter for no-load operation detection, the preset fluctuation range may be set to ±5% of a stable vaporization current value. When the vaporization parameters acquired in the fourth preset sampling time period all fall within the preset fluctuation range, it is determined that the vaporizer is currently in the stable vaporization stage, and sampling can be continued to calculate the sample mean.

It is to be understood that, the steps in FIG. 2, FIG. 3, and FIG. 5 are sequentially displayed as indicated by arrows, but the steps are not necessarily sequentially performed in an order indicated by the arrows. Unless otherwise explicitly specified in this specification, execution of the steps is not strictly limited, and the steps may be performed in other sequences. In addition, at least some steps in FIG. 2 to FIG. 4, and FIG. 5 may include a plurality of steps or a plurality of stages, and these steps or stages are not necessarily performed at a same time instant, but may be performed at different time instants. The steps or stages are not necessarily performed in sequence, but may be performed by turn or alternately with other steps or at least part of steps or stages in other steps.

The embodiments of this application further provide a no-load operation detection apparatus. As shown in FIG. 6, the no-load operation detection apparatus is applied to a vaporizer, and includes:

a variance calculation module 20, configured to obtain vaporization parameters of a vaporization piece in real time, and calculate a variance between the vaporization parameters and a sample mean, where the sample mean is a value capable of representing a vaporization parameter level of a stable vaporization stage of the vaporizer; and

a no-load operation determination module 40, configured to determine that the vaporization piece is operated without load if the variance is greater than a preset variance value, where the preset variance value is used for distinguishing a vaporization parameter fluctuation caused by vaporization operation without load and a vaporization parameter fluctuation caused by another operation other than vaporization without load.

In an embodiment, the variance calculation module 20 includes:
a multi-sampling variance calculation unit 22, configured to obtain vaporization parameters in a first preset sampling time period, and calculate a variance between vaporization parameters at sampling moments and the sample mean.

A second preset sampling time period includes a plurality of independent first preset sampling time periods, and the no-load operation determination module 40 includes: a variance mean obtaining unit 42, configured to calculate an average value of variances corresponding to vaporization parameters sampled in each of the first preset sampling time periods of the second preset sampling time period; and
a first no-load operation determining unit 44, configured to determine that the vaporization piece is operated without load if the average value of the variances is greater than the preset variance value.

In an embodiment, when the functional units of the no-load operation detection apparatus perform the steps, the used vaporization parameter includes a vaporization current and/or vaporization voltage peak-to-peak value and/or vaporization power.

In an embodiment, the no-load operation determination module 40 includes:

a first frequency sweep curve obtaining unit 46, configured to obtain an actual frequency sweep curve of the vaporization piece in a frequency sweep stage if the variance is greater than the preset variance value corresponding to the vaporization parameter, where the actual frequency sweep curve is data that reflects a change of the vaporization parameter when the vaporization piece works within a preset frequency range; and

a second no-load operation determining unit 48, configured to determine that the vaporization piece is operated without load if the actual frequency sweep curve does not match a preset frequency sweep curve, where the preset frequency sweep curve is a logic curve model of a change of a current when the vaporization piece works within the preset frequency range.

In an embodiment, the no-load operation determination module 40 includes:

a second frequency sweep curve obtaining unit 45, configured to obtain an actual frequency sweep curve of the vaporization piece in a frequency sweep stage if the average value of the variances is greater than the preset variance value corresponding to the vaporization parameter, where the actual frequency sweep curve is data that reflects a change of the vaporization parameter when the vaporization piece works within a preset frequency range; and

a second no-load operation determining unit 48, configured to determine that the vaporization piece is operated without load if the actual frequency sweep curve does not match a preset frequency sweep curve, where the preset frequency sweep curve is a logic curve model of a change of a current when the vaporization piece works within the preset frequency range.

In an embodiment, the no-load operation detection apparatus further includes:
a sample mean obtaining module 10, configured to obtain vaporization parameters of the stable vaporization stage, and determine the sample mean according to the vaporization parameters of the stable vaporization stage.
In an embodiment, the sample mean obtaining module 10 includes:

a stable vaporization parameter obtaining unit 12, configured to sample a plurality of vaporization parameters in a third preset sampling time period of the stable vaporization stage; and

a sample mean determination unit 14, configured to calculate an average value of the plurality of vaporization parameters, and use the average value as the sample mean. In an embodiment, the no-load operation detection apparatus further includes:
a fourth-preset-sampling-time-period vaporization parameter acquisition module 1, configured to obtain vaporization parameters in a fourth preset sampling time period; and a stable vaporization determination module 3, configured to determine that the vaporizer is in the stable vaporization stage if the vaporization parameters in the fourth preset sampling time period change within a preset fluctuation range.

For a specific limitation on the no-load operation detection apparatus, refer to the limitation on the no-load operation detection method above. Details are not described herein again. The modules in the no-load operation detection apparatus may be implemented entirely or partially by software, hardware, or a combination thereof. The foregoing modules may be built in or independent of a processor of a computer device in a hardware form, or may be stored in a memory of the computer device in a software form, so that the processor invokes and performs an operation corresponding to each of the foregoing modules. It should be noted that, in this embodiment of this application, the module division is an example, and is merely logical function division, and there may be other division manners during actual application.
In addition, the embodiments of this application further provide a no-load operation protection method. As shown in FIG. 7, the method is applied to a vaporizer, and includes the above no-load operation detection method; and the no-load operation protection method further includes the following step.
S900: Perform a no-load operation protection action if determining that the vaporization piece is operated without load.
The no-load operation protection action is an action that can remind a user to stop no-load operation of the vaporizer or can directly stop no-load operation of the vaporizer. For example, the no-load operation protection action may be to control a sound and light alarm set on the vaporizer to work, to remind the user to add test liquid or turn off the vaporizer; and may also be to send information for vaporization operation without load to a user terminal, such as a mobile phone of the user, to remind the user to change a working state of the vaporizer. In another example, the no-load operation protection action may also be to open up a communication pipeline between a liquid storage member and a vaporization

# EP 4 137 237 B1

tank in which the vaporization piece is located, for example, open a valve set in the pipeline, so that the test liquid stored in the liquid storage member can enter the vaporization tank in time, to change a state of no-load operation of the vaporization piece, thereby protecting the vaporization piece. In another example, the no-load operation protection action may also be to control the vaporizer to shut down, for example, control a switch connected in series with a channel of a power supply and a vaporizer controller to be disconnected, to stop supplying power to the vaporizer, so that the vaporization piece stops working, thereby protecting various components in the vaporizer from damage and avoiding safety accidents caused by long-term no-load operation.

As for the implementation process of no-load operation of the vaporization piece, reference may be made to descriptions in the above embodiments of the no-load operation detection method, and details are not described herein.

The embodiments of this application further provide a no-load operation protection apparatus. As shown in FIG. 6, the apparatus includes:

a variance calculation module 20, configured to obtain vaporization parameters of a vaporization piece in real time, and calculate a variance between the vaporization parameters and a sample mean, where the sample mean is a value capable of representing a vaporization parameter level of a stable vaporization stage of the vaporizer;

a no-load operation determination module 40, configured to determine that the vaporization piece is operated without load if the variance is greater than a preset variance value, where the preset variance value is used for distinguishing a vaporization parameter fluctuation caused by vaporization operation without load and a vaporization parameter fluctuation caused by another operation other than vaporization without load, and

a no-load operation protection execution module 90, configured to perform a no-load operation protection action if it is determined that the vaporization piece is operated without load.

For a specific limitation on the no-load operation protection apparatus, refer to the limitation on the no-load operation protection method and the no-load operation detection method above. Details are not described herein again. The modules in the above no-load operation protection apparatus may be implemented entirely or partially by software, hardware, or a combination thereof. The foregoing modules may be built in or independent of a processor of a computer device in a hardware form, or may be stored in a memory of the computer device in a software form, so that the processor invokes and performs an operation corresponding to each of the foregoing modules. It should be noted that, in this embodiment of this application, the module division is an example, and is merely logical function division, and there may be other division manners during actual application. A person skilled in the art should understand that, the no-load operation protection apparatus may implement any of beneficial effects of the above no-load operation detection apparatus.

In addition, the embodiments of this application further provide a vaporizer. As shown in FIG. 8, the vaporizer includes a vaporization piece 820, a sampling circuit 840, and a controller 860. The vaporization piece 820 is configured to vaporize to-be-vaporized liquid. The sampling circuit 840 is configured to sample vaporization parameters of the vaporization piece 820. The controller 860 is electrically connected to the vaporization piece 820 and the sampling circuit 840 respectively, and is configured to drive the vaporization piece 820 to vibrate, so that the vaporization piece 820 vaporizes the to-be-vaporized liquid; and the controller 860 is further configured to perform the steps of the no-load operation detection method and/or the steps of the no-load operation protection method.

By performing the steps in the above method embodiments and using variances to calculate a difference between an increased bubble signal and a no-load operation signal, the vaporizer equipped with the above controller 860 may improve the accuracy of no-load operation detection and avoid false detection of no-load operation caused when bubbles are generated, thereby improving the accuracy of performing the no-load operation protection action. For the specific implementation process, reference may be made to descriptions in the above method embodiments, and details are not repeated herein.

In an embodiment, as shown in FIG. 8, the controller 860 in the vaporizer may include: a drive circuit 862 and a processor 864. The drive circuit 862 is configured to drive the vaporization piece 820 to vibrate, so that the vaporization piece 820 vaporizes the to-be-vaporized liquid. The processor 864 is connected to the drive circuit 862, and is configured to regulate a signal outputted by the drive circuit 862 to the vaporization piece 820. The processor 864 can generate a drive signal to indicate the drive circuit 862 to drive the vaporization piece 820 to work at a resonant frequency, to generate a large amount of smoke and improve the use effect of the vaporizer. The drive signal may be a PWM drive signal.

In an embodiment, the vaporization parameter includes a vaporization current and/or vaporization voltage peak-to-peak value and/or vaporization power; and the sampling circuit 840 includes:

a first current sampling circuit 842, where an input end of the first current sampling circuit 842 is electrically connected to the vaporization piece 820, an output end of the first current sampling circuit 842 is connected to the processor 864, and the first current sampling circuit 842 is configured to sample the vaporization current; and/or

a vaporization voltage peak-to-peak value sampling circuit 844, where an input end of the vaporization voltage peak-to-peak value sampling circuit 844 is connected to an output end of the drive circuit 862, an output end of the vaporization voltage peak-to-peak value sampling circuit 844 is connected to the processor 864, and the vaporization voltage peak-to-peak value sampling circuit 844 is configured to sample the vaporization voltage peak-to-peak value; and/or

a voltage sampling circuit 846 and a second current sampling circuit 848, where an input end of the voltage sampling circuit 846 is connected to a vaporization power supply 880, an output end of the voltage sampling circuit 846 is connected to the processor 864, and the voltage sampling circuit 846 is configured to sample a vaporization input voltage; an input end of the second current sampling circuit 848 is configured to be connected in series between the power supply 880 and the drive circuit 862, and the second current sampling circuit 848 is configured to sample a vaporization input current; and the processor 864 is further configured to calculate the vaporization power according to the vaporization input voltage and the vaporization input current.

According to different selected vaporization parameters, different sampling circuits may be selected to sample the vaporization parameters, a variance calculation may be performed based on the vaporization parameters obtained by sampling, and no-load operation detection is implemented by comparing a variance and a preset variance value. In addition, when it is determined that the vaporization piece 820 is operated without load, a no-load operation protection action may be performed. When a plurality of vaporization parameters are acquired, it may be determined that the vaporization piece 820 is operated without load and the no-load operation protection action may be performed when a variance of any type of vaporization parameter is greater than a preset variance value of the vaporization parameter. The multiparameter detection manner can still ensure the effectiveness of the no-load operation detection of the vaporizer when a certain sampling circuit fails, thereby improving the reliability of no-load operation protection of the vaporizer. Various sampling circuits may feed back sampling signals thereof to the processor in the controller.

In an embodiment, the vaporizer further includes: the power supply 880, and the power supply 880 supplies power to the controller 860. The vaporizer may be configured with the power supply 880, to provide a matched working voltage for the drive circuit 862 and the processor 864 in the controller 860. The power supply 880 may include a rectification unit, a first voltage conversion unit, and a second voltage conversion unit. The rectification unit is configured to convert an external alternating current into a direct current, and transmit the direct current to the first voltage conversion unit and the second voltage conversion unit respectively. The first voltage conversion unit performs voltage conversion on the received direct current to generate a working voltage of the drive circuit 862, such as 12 V, and the second voltage conversion unit performs voltage conversion on the received direct current to generate a working voltage of the processor 864, such as 5 V, to meet requirements of the drive circuit 862 and the processor 864 for the working voltages. In an embodiment, the power supply 880 may also adopt a redundant design, to improve the working reliability of the vaporizer.

In an embodiment, the first voltage conversion unit may be an adjustable boost circuit, and the drive circuit drives the vaporization piece to work in a controller with the adjustable boost circuit and a processor.

In an embodiment, the vaporizer further includes: a vaporization tank 890 and a liquid storage member 891. The vaporization tank 890 is configured to accommodate the to-be-vaporized liquid, and the vaporization piece 820 is arranged in the vaporization tank 890. A liquid storage cavity is formed in the liquid storage member 891, the liquid storage cavity is configured to store the to-be-vaporized liquid, and the liquid storage cavity is in communication with the vaporization tank 890. Shapes of the vaporization tank 890 and the liquid storage member 891 are not limited as long as they can accommodate the to-be-vaporized liquid. Through the setting manner in which the vaporization tank 890 is in communication with the liquid storage member 891, it can be avoided that too much to-be-vaporized liquid in contact with the vaporization piece 820 influences the vaporization effect. As described in the above embodiment, when no-load operation is determined through no-load operation detection, a connection channel between the liquid storage member 891 and the vaporization tank 890 may be opened up, so that the to-be-vaporized liquid in the liquid storage member 891 enters the vaporization tank 890 for replenishment, thereby avoiding no-load operation while ensuring the vaporization effect.

In an embodiment, a computer device is provided. The computer device may be a terminal, and an internal structure diagram thereof may be shown in FIG. 9. The computer device includes a processor, a memory, a communication interface, a display screen, and an input apparatus that are connected by using a system bus. The processor of the computer device is configured to provide computing and control capabilities. The memory of the computer device includes a non-volatile storage medium and an internal memory. The non-volatile storage medium stores an operating system and a computer program. The internal memory provides an environment for running of the operating system and the computer program in the non-volatile storage medium. The communication interface of the computer device is configured to communicate with an external terminal in a wired or wireless manner, and the wireless manner may be implemented through WIFI, a carrier network, near field communication (NFC), or other technologies. The computer program is executed by the processor to implement a no-load operation detection method or a no-load operation protection method. The display screen of the computer device may be a liquid crystal display screen or an electronic ink display screen. The input apparatus of the computer device may be a touch layer covering the display screen, or may be a key, a trackball, or a touch pad disposed on a housing of the computer device, or may be an external keyboard, a touch pad, a mouse, or the like. For example, a touch screen is set on a housing of the vaporizer, and the preset variance value may be configured by the touch screen, to match a no-load operation detection requirement of specific types of to-be-vaporized liquid, thereby improving the accuracy of a no-load operation detection result.

A person skilled in the art may understand that, the structure shown in FIG. 9 is only a block diagram of a part of a structure related to a solution of this application, and does not limit the computer device to which the solution of this application is applied. Specifically, the computer device may include more or less members than those in the drawings, or include a combination of some members, or have a different arrangement of the members.

In an embodiment, a computer device is provided, including a memory and a processor, where the memory stores a computer program, and the processor, when executing the computer program, implements the following steps.

S200: Obtain vaporization parameters of a vaporization piece in real time, and calculate a variance between the vaporization parameters and a sample mean, where the sample mean is a value capable of representing a vaporization parameter level of a stable vaporization stage of the vaporizer.

S400: Determine that the vaporization piece is operated without load if the variance is greater than a preset variance value corresponding to the vaporization parameter, where the preset variance value is used for distinguishing a vaporization parameter fluctuation caused by vaporization operation without load and a vaporization parameter fluctuation caused by another operation other than vaporization without load.

In an embodiment, the processor, when executing the computer program, further implements the following steps.

Obtain vaporization parameters when the vaporization piece is operated without load, and calculate a no-load operation signal variance corresponding to the vaporization parameters when the vaporization piece is operated without load.

Obtain vaporization parameters of the vaporization piece when to-be-vaporized liquid is triggered to generate bubbles, and calculate a bubble signal variance corresponding to the vaporization parameters of the vaporization piece generated at the moment when the to-be-vaporized liquid is triggered to generate bubbles.

Select a value greater than the bubble signal variance and less than the no-load operation signal variance as the preset variance value.

In an embodiment, the processor, when executing the computer program, further implements the following step.

Select, after a plurality of times of trials, a value greater than bubble signal variances obtained from the trials and less than no-load operation signal variances obtained from the trials as the preset variance value.

In an embodiment, the processor, when executing the computer program, further implements the following step.

Obtain bubble signal variances and no-load operation signal variances when each type of the to-be-vaporized liquid is trialed, to determine a value that can meet a condition of being greater than the bubble signal variances obtained from the trials of various types of to-be-vaporized liquid and less than the no-load operation signal variances obtained from the trials of various types of to-be-vaporized liquid as the preset variance value in this scenario.

In an embodiment, the processor, when executing the computer program, further implements the following steps.

S220: Obtain vaporization parameters in a first preset sampling time period, and calculate a variance between vaporization parameters at sampling moments and the sample mean.

S420: Calculate an average value of variances corresponding to vaporization parameters sampled in each of the first preset sampling time periods of the second preset sampling time period. The second preset sampling time period includes at least one first preset sampling time period.

S440: Determine that the vaporization piece is operated without load if the average value of the variances is greater than the preset variance value.

In an embodiment, the processor, when executing the computer program, further implements the following steps.

S460: Obtain an actual frequency sweep curve of the vaporization piece in a frequency sweep stage if the variance is greater than the preset variance value corresponding to the vaporization parameter, where the actual frequency sweep curve is data that reflects a change of the vaporization parameter when the vaporization piece works within a preset frequency range.

S480: Determine that the vaporization piece is operated without load if the actual frequency sweep curve does not match a preset frequency sweep curve, where the preset frequency sweep curve is a logic curve model of a change of a current when the vaporization piece works within the preset frequency range.

In an embodiment, the processor, when executing the computer program, further implements the following step.

S 100: Obtain vaporization parameters of the stable vaporization stage, and determine the sample mean according to the vaporization parameters of the stable vaporization stage. In an embodiment, the processor, when executing the computer program, further implements the following steps.

S 120: Sample a plurality of vaporization parameters in a third preset sampling time period of the stable vaporization stage. The third preset sampling time period is a time period in the stable vaporization stage, and the time period may be adaptively selected by pre-configuring a number of times of sampling and a sampling frequency.

S 140: Calculate an average value of the plurality of vaporization parameters, and use the average value as the sample mean.

In an embodiment, the processor, when executing the computer program, further implements the following steps.

S 10: Obtain vaporization parameters in a fourth preset sampling time period.

S30: Determine that the vaporizer is in the stable vaporization stage if the vaporization parameters in the fourth preset sampling time period change within a preset fluctuation range.

In an embodiment, a computer-readable storage medium is provided, storing a computer program, and the computer program, when executed by a processor, implements the following steps.

S200: Obtain vaporization parameters of a vaporization piece in real time, and calculate a variance between the vaporization parameters and a sample mean, where the sample mean is a value capable of representing a vaporization parameter level of a stable vaporization stage of the vaporizer.

S400: Determine that the vaporization piece is operated without load if the variance is greater than a preset variance value corresponding to the vaporization parameter, where the preset variance value is used for distinguishing a vaporization parameter fluctuation caused by vaporization operation without load and a vaporization parameter fluctuation caused by another operation other than vaporization without load.

A person of ordinary skill in the art may understand that some or all procedures in the foregoing method embodiments may be implemented by a computer program instructing related hardware. The computer program may be stored in a non-volatile computer-readable storage medium, and when the computer program is executed, the procedures of the foregoing method embodiments may be performed. Any reference to a memory, a storage, a database, or another medium used in the embodiments provided in this application may include at least one of a non-volatile memory and a volatile memory. The non-volatile memory may include a read-only memory (ROM), a magnetic tape, a floppy disk, a flash memory, an optical memory, and the like. The volatile memory may include a random access memory (RAM) or an external cache. For the purpose of description instead of limitation, the RAM is available in a plurality of forms, such as a static RAM (SRAM) or a dynamic RAM (DRAM).

In description of this specification, description of reference terms such as "some embodiment", "other embodiments", or "ideal embodiments" means that specific features, structures, materials, or features described in conjunction with the embodiment or example are included in at least one embodiment or example of the present invention. In this specification, schematic descriptions of the foregoing terms do not necessarily point at a same embodiment or example.

The above methods, apparatuses and vaporizer provided in the embodiments of this application may be applied to medical, aesthetic and other fields, and in particular, to a scenario of using liquid sols such as essences that are prone to bubbles during vaporization. In the above solution provided in the embodiments of this application, high-precision no-load operation detection of the above methods, apparatuses and vaporizer can be ensured, thereby avoiding frequent shutdown of the vaporizer caused by an interference of bubble signals. Based on this, the working stability of the vaporizer and user experience can be improved.

**Claims**

1. A no-load operation detection method, being applied to a vaporizer, the method comprising:

   obtaining (S200) vaporization parameters of a vaporization piece in real time, and calculating a variance between the vaporization parameters and a sample mean, the sample mean being a value representing a vaporization parameter level of a stable vaporization stage of the vaporizer; and
   determining (S400) that the vaporization piece is operated without load if the variance is greater than a preset variance value corresponding to the vaporization parameter, the preset variance value being used for distinguishing a vaporization parameter fluctuation caused by vaporization operation without load and a vaporization parameter fluctuation caused by another operation other than vaporization without load;
   wherein the preset variance value is obtained by:

      obtaining vaporization parameters when the vaporization piece is no-load operation, and calculating a no-load operation signal variance corresponding to the vaporization parameters when the vaporization piece is no-load operation;
      obtaining vaporization parameters of the vaporization piece when to-be-vaporized liquid is triggered to generate bubbles, and calculating a bubble signal variance corresponding to the vaporization parameters of the vaporization piece generated at the moment when the to-be-vaporized liquid is triggered to generate bubbles; and
      selecting a value greater than the bubble signal variance and less than the no-load operation signal variance as the preset variance value.

2. The no-load operation detection method of claim 1, wherein obtaining (S200) vaporization parameters of the vaporization piece in real time, and calculating the variance between the vaporization parameters and the sample mean comprises: obtaining (S220) vaporization parameters in a first preset sampling time period, and calculating a variance between vaporization parameters at sampling moments and the sample mean, and

wherein a second preset sampling time period comprises a plurality of independent first preset sampling time periods, and determining (S400) that the vaporization piece is operated without load if the variance is greater than the preset variance value corresponding to the vaporization parameter comprises:

calculating (S420) an average value of variances corresponding to vaporization parameters sampled in each of the first preset sampling time periods of the second preset sampling time period; and

determining (S440) that the vaporization piece is operated without load if an average value of the variances is greater than the preset variance value.

3. The no-load operation detection method of claim 1, wherein the vaporization parameter comprises a vaporization current and/or vaporization voltage peak-to-peak value and/or vaporization power.

4. The no-load operation detection method of claim 1, wherein determining (S400) that the vaporization piece is operated without load if the variance is greater than the preset variance value corresponding to the vaporization parameter comprises:

obtaining (S460) an actual frequency sweep curve of the vaporization piece in a frequency sweep stage if the variance is greater than the preset variance value corresponding to the vaporization parameter, the actual frequency sweep curve being data that reflects a change of the vaporization parameter when the vaporization piece works within a preset frequency range; and

determining (S480) that the vaporization piece is operated without load if the actual frequency sweep curve does not match a preset frequency sweep curve, the preset frequency sweep curve being a logic curve model of a change of a current when the vaporization piece works within the preset frequency range.

5. The no-load operation detection method of any one of claims 1 to 4, further comprising:
obtaining (S100) vaporization parameters of the stable vaporization stage, and determining the sample mean of the vaporization parameters of the stable vaporization stage.

6. The no-load operation detection method of claim 5, wherein obtaining (S100) vaporization parameters of the stable vaporization stage, and determining the sample mean of the vaporization parameters of the stable vaporization stage comprises:

sampling (S120) a plurality of vaporization parameters in a third preset sampling time period of the stable vaporization stage; and

calculating (S140) an average value of the plurality of vaporization parameters, and using the average value as the sample mean.

7. The no-load operation detection method of claim 5, wherein before obtaining (S100) vaporization parameters of the stable vaporization stage, the method further comprises:

obtaining (S10) vaporization parameters in a fourth preset sampling time period; and

determining (S30) that the vaporizer is in the stable vaporization stage if the vaporization parameters in the fourth preset sampling time period change within a preset fluctuation range.

8. A no-load operation detection apparatus, being applied to a vaporizer, the apparatus comprising:

a variance calculation module (20) configured to obtain vaporization parameters of a vaporization piece in real time, and to calculate a variance between the vaporization parameters and a sample mean, the sample mean being a value representing a vaporization parameter level of a stable vaporization stage of the vaporizer; and

a no-load operation determination module (40) configured to determine that the vaporization piece is operated without load if the variance is greater than a preset variance value, the preset variance value being used for distinguishing a vaporization parameter fluctuation caused by vaporization operation without load and a vaporization parameter fluctuation caused by another operation other than vaporization without load;

wherein the preset variance value is obtained by:

obtaining vaporization parameters when the vaporization piece is no-load operation, and calculating a no-load operation signal variance corresponding to the vaporization parameters when the vaporization piece is no-load operation;

obtaining vaporization parameters of the vaporization piece when to-be-vaporized liquid is triggered to generate bubbles, and calculating a bubble signal variance corresponding to the vaporization parameters of the vaporization piece generated at the moment when the to-be-vaporized liquid is triggered to generate bubbles; and

selecting a value greater than the bubble signal variance and less than the no-load operation signal variance as the preset variance value.

9.  A vaporizer comprising:

a vaporization piece (140) configured to vaporize to-be-vaporized liquid;
a sampling circuit (160) configured to sample vaporization parameters of the vaporization piece (140); and
a controller (120) electrically connected to the vaporization piece (140) and the sampling circuit (160), respectively, the controller (120) being configured to:

drive the vaporization piece (140) to vibrate such that the vaporization piece (140) vaporizes the to-be-vaporized liquid, and
perform the steps of the no-load operation detection method of any one of claims 1 to 7.

10. The vaporizer of claim 9, wherein the controller (120) comprises:

a drive circuit (862) configured to drive the vaporization piece (140) to vibrate such that the vaporization piece (140) vaporizes the to-be-vaporized liquid; and
a processor (864) connected to the drive circuit (862), the processor (864) being configured to regulate a signal outputted by the drive circuit (862) to the vaporization piece (140).

11. The vaporizer of claim 10, wherein the vaporization parameter comprises a vaporization current and/or vaporization voltage peak-to-peak value and/or vaporization power, and
wherein the sampling circuit (160) comprises:

a first current sampling circuit (842), an input end of the first current sampling circuit (842) being electrically connected to the vaporization piece (140), an output end of the first current sampling circuit (842) being connected to the processor (864), and the first current sampling circuit (842) being configured to sample the vaporization current; and/or
a vaporization voltage peak-to-peak value sampling circuit (844), an input end of the vaporization voltage peak-to-peak value sampling circuit (844) being connected to an output end of the drive circuit (862), an output end of the vaporization voltage peak-to-peak value sampling circuit (844) being connected to the processor (864), and the vaporization voltage peak-to-peak value sampling circuit (844) being configured to sample the vaporization voltage peak-to-peak value; and/or
a voltage sampling circuit (846) and a second current sampling circuit (848), an input end of the voltage sampling circuit (846) being connected to a vaporization power supply (880), an output end of the voltage sampling circuit (846) being connected to the processor (864), and the voltage sampling circuit (846) being configured to sample a vaporization input voltage; an input end of the second current sampling circuit (848) being configured to be connected in series between the vaporization power supply (880) and the drive circuit (862) and the second current sampling circuit (848) being configured to sample a vaporization input current; and the processor (864) being further configured to calculate a vaporization power according to the vaporization input voltage and the vaporization input current.

12. The vaporizer of any one of claims 9 to 11, further comprising:

a vaporization tank (890) configured to accommodate the to-be-vaporized liquid, the vaporization piece (140) being arranged in the vaporization tank (890); and
a liquid storage member (891), a liquid storage cavity being formed in the liquid storage member (891), the liquid storage cavity being configured to store the to-be-vaporized liquid, and the liquid storage cavity being in communication with the vaporization tank (890).

**Patentansprüche**

1. Leerlaufbetrieberfassungsverfahren, das auf einen Verdampfer angewendet wird, wobei das Verfahren die Schritte aufweist:

   Erhalten (S200) von Verdampfungsparametern eines Verdampfungsteils in Echtzeit und Berechnen einer Varianz zwischen den Verdampfungsparametern und einem Abtastmittelwert, wobei der Abtastmittelwert ein Wert ist, der einen Verdampfungsparameterpegel einer stabilen Verdampfungsphase des Verdampfers darstellt; und

   Bestimmen (S400), dass das Verdampfungsteil im Leerlauf betrieben wird, wenn die Varianz größer ist als ein auf den Verdampfungsparameter bezogener voreingestellter Varianzwert, wobei der voreingestellte Varianzwert verwendet wird, um eine durch eine Verdampfungsbetrieb im Leerlauf verursachten Verdampfungsparameterfluktuation von einer Verdampfungsparameterfluktuation zu unterscheiden, die durch einen von einer Verdampfung im Leerlaufbetrieb verschiedenen Betrieb verursacht wird,

   wobei der voreingestellte Varianzwert erhalten wird durch:

   Erhalten von Verdampfungsparametern, wenn das Verdampfungsteil im Leerlauf betrieben wird, und Berechnen einer auf die Verdampfungsparameter bezogenen Leerlaufbetriebsignalvarianz, wenn das Verdampfungsteil im Leerlauf betrieben wird;

   Erhalten von Verdampfungsparametern des Verdampfungsteils, wenn zu verdampfende Flüssigkeit zur Blasenbildung angeregt wird, und Berechnen einer Blasensignalvarianz, die auf Verdampfungsparameter bezogen ist, die in dem Moment erzeugt werden, wenn die zu verdampfende Flüssigkeit zur Erzeugung von Blasen angeregt wird; und

   Auswählen eines Werts, der größer ist als die Blasensignalvarianz und kleiner als die Leerlaufbetriebsignalvarianz, als den voreingestellten Varianzwert.

2. Verfahren nach Anspruch 1, wobei das Erhalten (S200) von Verdampfungsparametern des Verdampfungsteils in Echtzeit und das Berechnen der Varianz zwischen den Verdampfungsparametern und dem Abtastmittelwert aufweist: Erhalten (S220) von Verdampfungsparametern in einer ersten voreingestellten Abtastzeitdauer und Berechnen einer Varianz zwischen Verdampfungsparametern zu Abtastzeitpunkten und dem Abtastmittelwert, und wobei eine zweite voreingestellte Abtastzeitdauer eine Vielzahl von unabhängigen ersten voreingestellten Abtastzeitdauern aufweist, und wobei das Bestimmen (S400), dass das Verdampfungsteil im Leerlauf betrieben wird, wenn die Varianz größer ist als der auf den Verdampfungsparameter bezogene voreingestellte Varianzwert, aufweist:

   Berechnen (S420) eines Durchschnittswerts von Varianzen, die auf Verdampfungsparameter bezogen sind, die in jeder der ersten voreingestellten Abtastzeitdauern der zweiten voreingestellten Abtastzeitdauer abgetastet werden; und

   Bestimmen (S440), dass das Verdampfungsteil im Leerlauf betrieben wird, wenn ein Durchschnittswert der Varianzen größer ist als der voreingestellte Varianzwert.

3. Verfahren nach Anspruch 1, wobei der Verdampfungsparameter einen Verdampfungsstrom und/oder einen Verdampfungsspannungswert zwischen Verdampfungsspannungsspitzen und/oder eine Verdampfungsleistung aufweist.

4. Verfahren nach Anspruch 1, wobei das Bestimmen (S400), dass das Verdampfungsteil im Leerlauf betrieben wird, wenn die Varianz größer ist als der auf den Verdampfungsparameter bezogene voreingestellte Varianzwert, aufweist:

   Erhalten (S460) einer tatsächlichen Frequenz-Sweep-Kurve des Verdampfungsteils in einer Frequenz-Sweep-Phase, wenn die Varianz größer ist als der auf den Verdampfungsparameter bezogene voreingestellte Varianzwert, wobei die tatsächliche Frequenz-Sweep-Kurve Daten sind, die eine Änderung des Verdampfungsparameters widerspiegeln, wenn das Verdampfungsteil innerhalb eines voreingestellten Frequenzbereichs arbeitet; und

   Bestimmen (S480), dass das Verdampfungsteil im Leerlauf betrieben wird, wenn die tatsächliche Frequenz-Sweep-Kurve nicht mit einer voreingestellten Frequenz-Sweep-Kurve übereinstimmt, wobei die voreingestellte Frequenz-Sweep-Kurve ein logisches Kurvenmodell einer Stromänderung ist, wenn das Verdampfungsteil innerhalb des voreingestellten Frequenzbereichs arbeitet.

5. Verfahren nach einem der Ansprüche 1 bis 4, ferner mit:
   Erhalten (S100) von Verdampfungsparametern der stabilen Verdampfungsphase und Bestimmen des Abtastmittelwerts der Verdampfungsparameter der stabilen Verdampfungsphase.

6. Verfahren nach Anspruch 5, wobei das Erhalten (S100) von Verdampfungsparametern der stabilen Verdampfungsphase und das Bestimmen des Abtastmittelwerts der Verdampfungsparameter der stabilen Verdampfungsphase aufweist:

Abtasten (S120) einer Vielzahl von Verdampfungsparametern in einer dritten voreingestellten Abtastzeitdauer der stabilen Verdampfungsphase; und
Berechnen (S140) eines Durchschnittswerts der Vielzahl von Verdampfungsparametern und Verwenden des Durchschnittswerts als den Abtastm ittelwert.

7. Verfahren nach Anspruch 5, wobei das Verfahren vor dem Erhalten (S100) von Verdampfungsparametern der stabilen Verdampfungsphase ferner aufweist:

Erhalten (S10) von Verdampfungsparametern in einer vierten voreingestellten Abtastzeitdauer; und
Bestimmen (S30), dass sich der Verdampfer in der stabilen Verdampfungsphase befindet, wenn sich die Verdampfungsparameter in der vierten voreingestellten Abtastzeitdauer innerhalb eines voreingestellten Fluktuationsbereichs ändern.

8. Leerlaufbetrieberfassungsvorrichtung, die auf einen Verdampfer angewendet wird, wobei die Vorrichtung aufweist:

ein Varianzberechnungsmodul (20), das dafür konfiguriert ist, Verdampfungsparameter eines Verdampfungsteils in Echtzeit zu erhalten und eine Varianz zwischen den Verdampfungsparametern und einem Abtastmittelwert zu berechnen, wobei der Abtastmittelwert ein Wert ist, der einen Verdampfungsparameterpegel einer stabilen Verdampfungsphase des Verdampfers darstellt; und
ein Leerlaufbetriebbestimmungsmodul (40), das dafür konfiguriert ist, zu bestimmen, dass das Verdampfungsteil im Leerlauf betrieben wird, wenn die Varianz größer ist als ein voreingestellter Varianzwert, wobei der voreingestellte Varianzwert zum Unterscheiden einer Verdampfungsparameterfluktuation, die durch einen Verdampfungsbetrieb im Leerlauf verursacht wird, und einer Verdampfungsparameterfluktuation, die durch einen von einem Verdampfungsbetrieb im Leerlauf verschiedenen Betrieb verursacht wird, verwendet wird,
wobei der voreingestellte Varianzwert erhalten wird durch:

Erhalten von Verdampfungsparametern, wenn das Verdampfungsteil im Leerlauf betrieben wird, und Berechnen einer auf die Verdampfungsparameter bezogenen Leerlaufbetriebssignalvarianz, wenn das Verdampfungsteil im Leerlauf betrieben wird;
Erhalten von Verdampfungsparametern des Verdampfungsteils, wenn zu verdampfende Flüssigkeit zur Erzeugung von Blasen angeregt wird, und Berechnen einer Blasensignalvarianz, die auf die Verdampfungsparameter des Verdampfungsteils bezogen sind, die in dem Moment erzeugt werden, in dem die zu verdampfende Flüssigkeit zur Erzeugung von Blasen angeregt wird; und
Auswählen eines Werts, der größer ist als die Blasensignalvarianz und kleiner als die Leerlaufbetriebssignalvarianz, als den voreingestellten Varianzwert.

9. Verdampfer, mit:

einem Verdampfungsteil (140), das dafür konfiguriert ist, zu verdampfende Flüssigkeit zu verdampfen;
einer Abtastschaltung (160), die dafür konfiguriert ist, Verdampfungsparameter des Verdampfungsteils (140) abzutasten; und
einer Steuereinheit (120), die jeweils elektrisch mit dem Verdampfungsteil (140)und der Abtastschaltung (160) verbunden ist, wobei die Steuereinheit (120) dafür konfiguriert ist:

das Verdampfungsteil (140) so anzutreiben, dass es vibriert, so dass das Verdampfungsteil (140) die zu verdampfende Flüssigkeit verdampft; und
die Schritte des Leerlaufbetrieberfassungsverfahrens nach einem der Ansprüche 1 bis 7 auszuführen.

10. Verdampfer nach Anspruch 9, wobei die Steuereinheit (120) aufweist:

eine Antriebsschaltung (862), die dafür konfiguriert ist, das Verdampfungsteil (140) so in Vibration zu versetzen, dass das Verdampfungsteil (140) die zu verdampfende Flüssigkeit verdampft; und

einen mit der Antriebsschaltung (862) verbundenen Prozessor (864), wobei der Prozessor (864) dafür konfiguriert ist, ein von der Antriebsschaltung (862) an das Verdampfungsteil (140) ausgegebenes Signal zu regulieren.

11. Verdampfer nach Anspruch 10, wobei der Verdampfungsparameter einen Verdampfungsstrom und/oder einen Verdampfungsspannungswert zwischen Verdampfungsspannungsspitzen und/oder eine Verdampfungsleistung aufweist, und
wobei die Abtastschaltung (160) aufweist:

eine erste Stromabtastschaltung (842), wobei ein Eingangsende der ersten Stromabtastschaltung (842) elektrisch mit dem Verdampfungsteil (140) verbunden ist, ein Ausgangsende der ersten Stromabtastschaltung (842) mit dem Prozessor (864) verbunden ist und die erste Stromabtastschaltung (842) dafür konfiguriert ist, den Verdampfungsstrom abzutasten; und/oder
eine Verdampfungsspannungsspitzenwertabtastschaltung (844), wobei ein Eingangsende der Verdampfungsspannungsspitzenwertabtastschaltung (844) mit einem Ausgangsende der Antriebsschaltung (862) verbunden ist, ein Ausgangsende der Verdampfungsspannungsspitzenwertabtastschaltung (844) mit dem Prozessor (864) verbunden ist und die Verdampfungsspannungsspitzenwertabtastschaltung (844) dafür konfiguriert ist, den Spannungswert zwischen Verdampfungsspannungsspitzen abzutasten; und/oder
eine Spannungsabtastschaltung (846) und eine zweite Stromabtastschaltung (848), wobei ein Eingangsende der Spannungsabtastschaltung (846) mit einer Verdampfungsstromversorgung (880) verbunden ist, ein Ausgangsende der Spannungsabtastschaltung (846) mit dem Prozessor (864) verbunden ist und die Spannungsabtastschaltung (846) dafür konfiguriert ist, eine Verdampfungseingangsspannung abzutasten, wobei ein Eingangsende der zweiten Stromabtastschaltung (848) dafür konfiguriert ist, in Reihe zwischen der Verdampfungsstromversorgung (880) und der Antriebsschaltung (862) geschaltet zu werden, und wobei die zweite Stromabtastschaltung (848) dafür konfiguriert ist, einen Verdampfungseingangsstrom abzutasten, und wobei der Prozessor (864) ferner dafür konfiguriert ist, eine Verdampfungsleistung gemäß der Verdampfungseingangsspannung und dem Verdampfungseingangsstrom zu berechnen.

12. Verdampfer nach einem der Ansprüche 9 bis 11, ferner mit:

einem Verdampfungstank (890), der dafür konfiguriert ist, die zu verdampfende Flüssigkeit aufzunehmen, wobei das Verdampfungsteil (140) im Verdampfungstank (890) angeordnet ist; und
einem Flüssigkeitsspeicherelement (891), wobei im Flüssigkeitsspeicherelement (891) ein Flüssigkeitsspeicherhohlraum ausgebildet ist, wobei der Flüssigkeitsspeicherhohlraum dafür konfiguriert ist, die zu verdampfende Flüssigkeit zu speichern, und wobei der Flüssigkeitsspeicherhohlraum mit dem Verdampfungstank (890) kommuniziert.

## Revendications

1. Méthode de détection d'un fonctionnement sans charge,
appliquée à un vaporisateur, la méthode comprenant les étapes consistant à :

obtenir (S200) des paramètres de vaporisation d'une pièce de vaporisation en temps réel, et calculer une variance entre les paramètres de vaporisation et une moyenne d'échantillon, la moyenne d'échantillon étant une valeur représentant un niveau de paramètres de vaporisation d'un stade de vaporisation stable du vaporisateur ; et
déterminer (S400) que la pièce de vaporisation fonctionne sans charge si la variance est supérieure à une valeur de variance prédéfinie correspondant au paramètre de vaporisation, la valeur de variance prédéfinie étant utilisée pour distinguer une fluctuation de paramètre de vaporisation amenée par un fonctionnement de vaporisation sans charge et une fluctuation de paramètre de vaporisation amenée par un fonctionnement de vaporisation sans charge et une fluctuation de paramètre de vaporisation amenée par un autre fonctionnement différent de la vaporisation sans charge ;
dans laquelle la valeur de variance prédéfinie est obtenue en :

obtenant des paramètres de vaporisation lorsque la pièce de vaporisation est en fonctionnement sans charge, et en calculant une variance de signal de fonctionnement sans charge correspondant aux paramètres de vaporisation lorsque la pièce de vaporisation est en fonctionnement sans charge ;
obtenant des paramètres de vaporisation de la pièce de vaporisation lorsque le liquide à vaporiser est entraîné à générer des bulles, et en calculant une variance de signal de bulle correspondant aux paramètres de vaporisation de la pièce de vaporisation générée au moment où le liquide à vaporiser est entraîné à générer des bulles ; et

sélectionnant une valeur supérieure à la variance de signal de bulle et inférieure à la variance de signal de fonctionnement sans charge en tant que valeur de variance prédéfinie.

2. Méthode de détection de fonctionnement sans charge selon la revendication 1, dans laquelle les étapes consistant à obtenir (S200) des paramètres de vaporisation de la pièce de vaporisation en temps réel, et à calculer la variance entre les paramètres de vaporisation et la moyenne d'échantillon comprennent les étapes consistant à : obtenir (S220) des paramètres de vaporisation dans une première période d'échantillonnage prédéfinie, et calculer une variance entre les paramètres de vaporisation à des moments d'échantillonnage et la moyenne d'échantillon, et dans laquelle une seconde période d'échantillonnage prédéfinie comprend une pluralité de premières périodes d'échantillonnage prédéfinies indépendantes, et déterminer (S400) que la pièce de vaporisation fonctionne sans charge si la variance est supérieure à la valeur de variance prédéfinie correspondant au paramètre de vaporisation comprend les étapes consistant à :

calculer (S420) une valeur moyenne de variances correspondant à des paramètres de vaporisation échantillonnés dans chacune des premières périodes d'échantillonnage prédéfinies de la seconde période d'échantillonnage prédéfinie ; et

déterminer (S440) que la pièce de vaporisation fonctionne sans charge si une valeur moyenne des variances est supérieure à la valeur de variance prédéfinie.

3. Méthode de détection de fonctionnement sans charge selon la revendication 1, dans laquelle le paramètre de vaporisation comprend un courant de vaporisation et/ou une valeur de tension de vaporisation crête à crête et/ou une puissance de vaporisation.

4. Méthode de détection de fonctionnement sans charge selon la revendication 1, dans laquelle l'étape consistant à déterminer (S400) que la pièce de vaporisation fonctionne sans charge si la variance est supérieure à la valeur de variance prédéfinie correspondant au paramètre de vaporisation comprend les étapes consistant à :

obtenir (S460) une courbe de balayage en fréquence réelle de la pièce de vaporisation dans un stade de balayage en fréquence si la variance est supérieure à la valeur de variance prédéfinie correspondant au paramètre de vaporisation, la courbe de balayage en fréquence réelle étant des données qui reflètent une variation du paramètre de vaporisation lorsque la pièce de vaporisation fonctionne au sein d'une plage de fréquence prédéfinie ; et

déterminer (S480) que la pièce de vaporisation fonctionne sans charge si la courbe de balayage de fréquence réelle ne correspond pas à une courbe de balayage de fréquence prédéfinie, la courbe de balayage de fréquence prédéfinie étant un modèle de courbe logique de variation d'un courant lorsque la pièce de vaporisation fonctionne dans la plage de fréquence prédéfinie.

5. Méthode de détection de fonctionnement sans charge selon l'une quelconque des revendications 1 à 4, comprenant en outre l'étape consistant à :
obtenir (S 100) des paramètres de vaporisation du stade de vaporisation stable, et déterminer la moyenne d'échantillon des paramètres de vaporisation du stade de vaporisation stable.

6. Méthode de détection de fonctionnement sans charge selon la revendication 5, dans laquelle l'étape consistant à obtenir (S100) des paramètres de vaporisation du stade de vaporisation stable, et déterminer la moyenne d'échantillon des paramètres de vaporisation du stade de vaporisation stable comprend les étapes consistant à :

échantillonner (S120) une pluralité de paramètres de vaporisation dans une troisième période d'échantillonnage prédéfinie du stade de vaporisation stable ; et

calculer (S140) une valeur moyenne de la pluralité de paramètres de vaporisation, et utiliser la valeur moyenne en tant que moyenne d'échantillon.

7. Méthode de détection de fonctionnement sans charge selon la revendication 5, dans laquelle avant l'étape consistant à obtenir (S100) des paramètres de vaporisation du stade de vaporisation stable, la méthode comprend en outre l'étape consistant à :
obtenir (S10) les paramètres de vaporisation dans une quatrième période d'échantillonnage prédéfinie ; et déterminer (S30) que le vaporisateur est dans le stade de vaporisation stable si les paramètres de vaporisation de la quatrième période d'échantillonnage prédéfinie varient dans une plage de fluctuation prédéfinie.

8. Appareil de détection de fonctionnement sans charge, appliqué à un vaporisateur, l'appareil comprenant :

un module de calcul de variance (20) configuré pour obtenir des paramètres de vaporisation d'une pièce de vaporisation en temps réel, et pour calculer une variance entre les paramètres de vaporisation et une moyenne d'échantillon, la moyenne d'échantillon étant une valeur représentant un niveau de paramètres de vaporisation d'un stade de vaporisation stable du vaporisateur ; et

un module de détermination de fonctionnement sans charge (40) configuré pour déterminer que la pièce de vaporisation fonctionne sans charge si la variance est supérieure à une valeur de variance prédéfinie, la valeur de variance prédéfinie étant utilisée pour distinguer une fluctuation de paramètre de vaporisation amenée par un fonctionnement de vaporisation sans charge et une fluctuation de paramètre de vaporisation amenée par un fonctionnement de vaporisation sans charge et une fluctuation de paramètre de vaporisation amenée par un autre fonctionnement différent de la vaporisation sans charge ;

dans lequel la valeur de variance prédéfinie est obtenue en :

obtenant des paramètres de vaporisation lorsque la pièce de vaporisation est en fonctionnement sans charge, et en calculant une variance de signal de fonctionnement sans charge correspondant aux paramètres de vaporisation lorsque la pièce de vaporisation est en fonctionnement sans charge ;

obtenant des paramètres de vaporisation de la pièce de vaporisation lorsque le liquide à vaporiser est entraîné à générer des bulles, et en calculant une variance de signal de bulle correspondant aux paramètres de vaporisation de la pièce de vaporisation générée au moment où le liquide à vaporiser est entraîné à générer des bulles ; et

sélectionnant une valeur supérieure à la variance de signal de bulle et inférieure à la variance de signal de fonctionnement sans charge en tant que valeur de variance prédéfinie.

9. Vaporisateur comprenant :

une pièce de vaporisation (140) conçue pour vaporiser un liquide à vaporiser ;

un circuit d'échantillonnage (160) configuré pour échantillonner les paramètres de vaporisation de la pièce de vaporisation (140) ; et

un dispositif de commande (120) connecté électriquement à la pièce de vaporisation (140) et au circuit d'échantillonnage (160), respectivement, le dispositif de commande (120) étant configuré pour :

entraîner la pièce de vaporisation (140) à vibrer de telle sorte que la pièce de vaporisation (140) vaporise le liquide à vaporiser, et

réaliser les étapes de la méthode de détection de fonctionnement sans charge selon l'une quelconque des revendications 1 à 7.

10. Vaporisateur selon la revendication 9, dans lequel le dispositif de commande (120) comprend :

un circuit d'entraînement (862) configuré pour entraîner la pièce de vaporisation (140) à vibrer de telle sorte que la pièce de vaporisation (140) vaporise le liquide à vaporiser ; et

un processeur (864) connecté au circuit d'entraînement (862), le processeur (864) étant configuré pour réguler un signal émis par le circuit d'entraînement (862) à la pièce de vaporisation (140).

11. Vaporisateur selon la revendication 10, dans laquelle le paramètre de vaporisation comprend un courant de vaporisation et/ou une valeur de tension de vaporisation crête à crête et/ou une puissance de vaporisation, et dans lequel le circuit d'échantillonnage (160) comprend :

un premier circuit d'échantillonnage de courant (842), une extrémité d'entrée du premier circuit d'échantillonnage de courant (842) étant connectée électriquement à la pièce de vaporisation (140), une extrémité de sortie du premier circuit d'échantillonnage de courant (842) étant connectée au processeur (864), et le premier circuit d'échantillonnage de courant (842) étant configuré pour échantillonner le courant de vaporisation ; et/ou

un circuit d'échantillonnage de valeur de tension de vaporisation crête à crête (844), une extrémité d'entrée du circuit d'échantillonnage de valeur de tension de vaporisation crête à crête (844) étant connectée à une extrémité de sortie du circuit d'entraînement (862), une extrémité de sortie du circuit d'échantillonnage de valeur de tension de vaporisation crête à crête (844) étant connectée au processeur (864), et le circuit d'échantillonnage de valeur de tension de vaporisation crête à crête (844) étant configuré pour échantillonner la valeur de tension de vaporisation crête à crête ; et/ou

un circuit d'échantillonnage de tension (846) et un second circuit d'échantillonnage de courant (848), une extrémité d'entrée du circuit d'échantillonnage de tension (846) étant connectée à une alimentation électrique de vaporisation (880), une extrémité de sortie du circuit d'échantillonnage de tension (846) étant connectée au processeur (864), et le circuit d'échantillonnage de tension (846) étant configuré pour échantillonner une tension d'entrée de vaporisation ; une extrémité d'entrée du second circuit d'échantillonnage de courant (848) étant configurée pour être connectée en série entre l'alimentation électrique de vaporisation (880) et le circuit d'entraînement (862) et le second circuit d'échantillonnage de courant (848) étant configuré pour échantillonner un courant d'entrée de vaporisation ; et le processeur (864) étant en outre configuré pour calculer une puissance de vaporisation en fonction de la tension d'entrée de vaporisation et du courant d'entrée de vaporisation.

12. Vaporisateur selon l'une quelconque des revendications 9 à 11, comprenant en outre :

un réservoir de vaporisation (890) conçu pour accueillir le liquide à vaporiser, la pièce de vaporisation (140) étant agencée dans le réservoir de vaporisation (890) ; et

un élément de stockage de liquide (891), une cavité de stockage de liquide étant formée dans l'élément de stockage de liquide (891), la cavité de stockage de liquide étant conçue pour stocker le liquide à vaporiser, et la cavité de stockage de liquide étant en communication avec le réservoir de vaporisation (890).

120

Controller

140

Vaporization
piece

160

Sampling circuit

## FIG. 1

S100

Obtain vaporization parameters of the stable vaporization stage, and determine the sample mean
according to the vaporization parameters of the stable vaporization stage

S200

Obtain vaporization parameters of a vaporization piece in real time, and calculate a variance between
the vaporization parameters and a sample mean, where the sample mean is a value capable of
representing a vaporization parameter level of a stable vaporization stage of the vaporizer

S400

Determine that the vaporization piece is operated without load if the variance is greater than a preset
variance value corresponding to the vaporization parameter, where the preset variance value is used for
distinguishing a vaporization parameter fluctuation caused by vaporization operation without load and a
vaporization parameter fluctuation caused by another operation other than vaporization without load

## FIG. 2

S220

Obtain vaporization parameters in a first preset sampling time period, and calculate a variance between vaporization parameters at sampling moments and the sample mean

S420

Calculate an average value of variances corresponding to vaporization parameters sampled in each of the first preset sampling time periods of the second preset sampling time period

S440

Determine that the vaporization piece is operated without load if the average value of the variances is greater than the preset variance value

# FIG. 3

Bubble signal

Threshold 1

A1

Current average value

225
220
215
210
205
200
195
190

1  6  11  15  21  26  31  36  41  45  51  56  61  66  71  76  81  86  91  96  101  106  111  116

# FIG. 4a(1)

Variance calculation of bubble signal

A2

Current average value

900
800
700
600
500
400
300
200
100
0

1 4 7 1013161922252831343740434649525558616467707376798285889194 97

## FIG. 4a(2)

No-load operation signal

Threshold 1

B1

Current average value

225

220

215

210

205

200

195

190

1 6 11 16 21 26 31 36 41 46 51 56 61 66 71 76 81 86 91 96 101 106 111 116

## FIG. 4b(1)

FIG. 4b(2)

FIG. 4c

S10

Obtain vaporization parameters in a fourth preset sampling time period

S30

Determine that the vaporizer is in the stable vaporization stage if the vaporization parameters in the fourth preset sampling time period change within a preset fluctuation range

S120

Sample a plurality of vaporization parameters in a third preset sampling time period of the stable vaporization stage

S140

Calculate an average value of the plurality of vaporization parameters, and use the average value as the sample mean

S200

Obtain vaporization parameters of a vaporization piece in real time, and calculate a variance between the vaporization parameters and a sample mean, where the sample mean is a value capable of representing a vaporization parameter level of a stable vaporization stage of the vaporizer

S460

Obtain an actual frequency sweep curve of the vaporization piece in a frequency sweep stage if the variance is greater than the preset variance value corresponding to the vaporization parameter, where the actual frequency sweep curve is data that reflects a change of the vaporization parameter when the vaporization piece works within a preset frequency range

S480

Determine that the vaporization piece is operated without load if the actual frequency sweep curve does not match a preset frequency sweep curve, where the preset frequency sweep curve is a logic curve model of a change of a current when the vaporization piece works within the preset frequency range

# FIG. 5

Fourth-preset-sampling-time-period vaporization parameter acquisition module — 1

Stable vaporization determination module — 3

Stable vaporization parameter obtaining unit — 12

Sample mean determination unit — 14

— 10

Multi-sampling variance calculation unit — 22

— 20

No-load operation protection execution module — 40

Variance mean obtaining unit — 42

First no-load operation determining unit — 44

First frequency sweep curve obtaining unit — 46

Second no-load operation determining unit — 48

Second frequency sweep curve obtaining unit — 45

No-load operation protection execution module — 90

FIG. 6

S100

Obtain vaporization parameters of the stable vaporization stage, and determine the sample mean according to the vaporization parameters of the stable vaporization stage

S200

Obtain vaporization parameters of a vaporization piece in real time, and calculate a variance between the vaporization parameters and a sample mean, where the sample mean is a value capable of representing a vaporization parameter level of a stable vaporization stage of the vaporizer

S400

Determine that the vaporization piece is operated without load if the variance is greater than a preset variance value corresponding to the vaporization parameter, where the preset variance value is used for distinguishing a vaporization parameter fluctuation caused by vaporization operation without load and a vaporization parameter fluctuation caused by another operation other than vaporization without load

S900

Perform a no-load operation protection action if determining that the vaporization piece is operated without load

FIG. 7

FIG. 8

FIG. 9

**EP 4 137 237 B1**

**Patent documents cited in the description**

- EP 2554514 A1
- EP 3831228 A1